# EUROPEAN PATENT SPECIFICATION

(11) **EP 1 977 679 B1**
(45) Date of publication and mention of the grant of the patent: **18.08.2010**
(21) Application number: 08006881.0
(22) Date of filing: 04.04.2008
(51) Int. Cl.: A61B 1/01, A61B 1/273

(54) **Therapeutic method and therapeutic system using an overtube with balloons**
Therapeutisches Verfahren und therapeutisches System mit einem Überrohr mit Ballons
Procédé thérapeutique et système thérapeutique utilisant un sur-tube avec des ballons

(30) Priority: 04.04.2007 US 732660
(43) Date of publication of application: 08.10.2008
(73) Proprietor: Olympus Medical Systems Corp., Tokyo 151-0072 (JP)
(72) Inventor: Motai, Kousuke, Tokyo 151-0072 (JP); Onuki, Yoshio, Tokyo 151-0072 (JP); Kura, Yasuhito, Tokyo 151-0072 (JP)
(74) Representative: von Hellfeld, Axel

(56) References cited:
- EP-A- 1 654 977
- JP-A- 2002 282 268
- US-A- 5 400 770
- US-A1- 2007 015 965

## Description

### Background of the Invention

### (Technical field of the Invention)

The present invention relates to a therapeutic system which can be used in a therapeutic method that treats a lesion within complex tubular cavities, including the biliary duct, pancreatic duct, and duodenum, of an object being examined, with the help of an overtube equipped with a plurality of balloons.

### (Related Art)

Endoscopic examinations and treatments for biliary disease and pancreas disease, such as biliary tract cancer, pancreas cancer, cholelithiasis, and common bile duct stones, have been on the rapid progress. Compared to the conventional surgical treatments, these examinations and treatments are less invasive and of a less burden on patients. The techniques for these examinations and treatments include endoscopic retrograde cholangiopancreatography (ERCP) and Endoscopic sphincterotomy. These endoscopic examinations and treatments are on the progress for applications to postgastrectomy cases.

Under such circumstances, when the insertion tube of an endoscope is inserted into an object being examined, an overtube is used also. One example of such an overtube is shown by Japanese Patent Publication (Laid-open) No. 62(1987)-22623 which discloses an insertion assisting device for endoscopes. This assisting device is a cylindrical member which is a guide and allows the insertion tube to inserted therethrough, when the insertion tube is inserted into a body cavity of the object. At a distal-end-slde predetermined position of this cylindrical member, an opening is formed to communicate between the inside and outside of this member for allowing the endoscope to be passed therethrough. Further, at another predetermined side position which is closer to the distal end than the opening, the cylindrical member is equipped with a balloon which can be expanded and shrunk by supplying and draining fluid. Thus, when the balloon Is expanded at a desired position in a body cavity, the distal end of the overtube can be fixed to the wall of the body cavity.

However, since the above example adopts only one balloon, it is not always certain that the balloon provides a secure positional fixing. For example, the inner diameter of a duodenum may be inconsistent with the diameter of the balloon expanded. Furthermore, as only one point is given as the fixing point to the organ, the fixing may have a weaker resistance against peristaltic motions. Thus the positional fixing may not be assured in its reliability, possibly causing the overtube to move. To overcome this difficulty, one countermeasure is to raise the expansion rate of the balloon to obtain a higher strength of positional fixing. In this case, however, a burden imposed on the patient becomes higher as well. Under such a situation, it has been desired to have a more effective positional fixing function in using an overtube for endoscopes. In addition, how to effectively fix the position of an endoscope in treating pancreatic and biliary ducts of a Roux-en-Y gastrectomy patient has not been resolved yet.

On the other hand, a catheter or tube equipped with a balloon is known for guiding the catheter and fixing its position in the body.

For example, Japanese Utility Model Publication No. 56-26104 (Reference 1) discloses a structure in which, by way of example, three expandable balloons are loaded on a tube body at moderate intervals, and in practical use, each balloon functions as a member to perform various functions, such as preventing blood from coming into the stomach, stopping bleeding, and preventing blood from flowing back. In addition, Japanese Patent Laid-open Publication No. 8-299432 (Reference 2) discloses a suction tube with a stomach balloon and an esophagus balloon. The stomach balloon is formed to have a series of two spheres when being expanded, and can come into contact with the stomach wall that surrounds the cardia in the stomach. The contact area is smaller than the spherical balloon, thus being preferable when the suction tube is pulled out.

Further, Japanese Patent Laid-open Publication No. 2003-88495 (Reference 3) discloses an endoscopic balloon apparatus in which a flexible tube is provided with two balloons which can be expanded and shrunk and which are loaded at the distal end of the tube. Of the two balloons, the balloon located inside the stomach can be expanded larger than the balloon located outside the stomach at the mouth of the stomach. This makes the inside of the stomach expand reliably so that a stopper function is obtained at the mouth of the stomach.

Still further, Japanese Patent Laid-open Publication No. 2003-230629 (Reference 4) discloses a balloon catheter in which a plurality of balloons are serially connected to each other with flexible connections therebetween. This balloon catheter can be inserted into, for example, a cerebral blood vessel and used to place a stent therein. Of the plural balloons, the expansion rate of balloons located at parts other than a lesion can be controlled so as not to be expanded.

However, the balloon(s) employed by the balloon catheter and balloon apparatus according to the foregoing references 1 - 4 cannot be employed by an overtube. The reason is that such balloons cannot fix the position of the overtube itself in a body cavity or a tubular cavity of an object being examined.

US 5,400,770 discloses a surgical device for use with an endoscope comprising two balloons spaced apart in the axial direction of an elongate tubular sheath.

Patent Abstracts of Japan 2002-282268 also discloses a medical sheath for inserting an endoscope, wherein two balloons are spaced apart in the axial direction of the medical sheath.

### Summery of the Invention

The present invention has been made in consideration of the problem that the foregoing overtube for endoscopes has confronted, and has an object to provide an overtube for endoscopes which is able to be fixed easily and reliably, and a therapeutic system using the overtube in position. The object of the present invention is achieved by an overtube according to claim 1.

An exemplary overtube comprises: a flexible tubular portion in which an insertion channel is formed, an endoscope being inserted through the insertion channel; and a plurality of balloons loaded at plural positions on an outer surface of the flexible tubular portion, the balloons being expanded and shrunk selectively every use pattern, the positions being different from each other in an axial direction of the flexible tubular portion and being set to be fixed to an inner wall of either a body cavity or a tubular cavity of an object being examined at mutually different positions of the inner wall by expanding the balloons.

As another aspect of the present invention, there is provided an overtube comprising: a flexible tubular portion in which an insertion channel is formed, an endoscope being inserted through the insertion channel; and a plurality of balloons loaded at plural positions on an outer surface of the flexible tubular portion, the balloons being expanded and shrunk selectively every use pattern, being substantially equal in internal cubic volume to each other when being expanded, respectively, and including at least two balloons having mutually different lengths in the axial direction, the positions being different from each other in an axial direction of the flexible tubular portion and being set to be fixed to an inner wall of either a body cavity or a tubular cavity of an object being examined at mutually different positions of the inner wall by expanding the balloons.

In the overtube according to this mode, the plurality of balloons are loaded at plural positions on an outer surface of the flexible tubular portion and are expanded and shrunk, while the positions are set to be fixed to an inner wall of either a body cavity or a tubular cavity of an object being examined at mutually different positions of the inner wall by expanding the balloons.

A therapeutic system comprises: an endoscope; an overtube comprising an insertion channel through which the endoscope is inserted, a flexible tubular portion, and a plurality of balloons loaded at plural positions respectively on an outer surface of the flexible tubular portion, the balloons being selectively expanded and shrunk, the positions being different from each other in an axial direction of the flexible tubular portion and being set to be fixed to an inner wall of either a body cavity or a tubular cavity of an object being examined at mutually different positions of the inner wall by expanding the balloons; and a controller that selectively controls the expansion of the plurality of balloons.

A therapeutic method comprises the steps of: inserting an insertion tube of an endoscope through an insertion channel of an overtube equipped with a plurality of balloons; inserting orally the insertion tube of the endoscope, together with the overtube, until a duodenum of an object being examined located adjacently to a Vater's papilla of the duodenum; moving the overtube toward the Vater's papilla along the insertion tube of the endoscope so as to locate a distal end of the overtube adjacently to the Vater's papilla; expanding at least two balloons of the plurality of balloons so that the at least two balloons are fixed respectively to an inner wall of a first tubular cavity or body cavity and a second tubular cavity or body cavity both leading to the duodenum; and using the endoscope to diagnose and/or treat at least any one of a biliary duct, a pancreatic duct, and a neighboring part to the Vater's papilla, after expanding the at least two balloons to be positionally fixed.

### Brief Description of the Drawings

In the accompanying drawings:
Fig. 1 is a perspective view outlining the configuration of a therapeutic system according to a first embodiment of the present invention;
Fig. 2 is a side view taken along an A-A line in Fig. 1;
Fig. 3 is a sectional view taken along a B-B line in Fig. 1;
Fig. 4 is a partial sectional view explaining the positional relationship between a balloon and an air-supply channel;
Figs. 5 - 7 explain not only an endoscope, together with an overtube, inserted into an object being examined but also positional fixing with the balloons in the first embodiment;
Fig. 8 explains not only an endoscope, together with an overtube, inserted into an object being examined but also positional fixing with the balloons in a modification 1;
Figs. 9 - 14 explain not only an endoscope, together with an overtube, inserted into an object being examined but also positional fixing with the balloons in a modification 2;
Fig. 15 explains an overtube and a syringe serving as an air-supply device, both of which are according to a modification 3;
Fig. 16A is a side view showing an overtube according to a modification 4;
Fig. 16B is a sectional view taken along a C-C line in Fig. 16A;
Fig. 17 explains not only an endoscope, together with an overtube, inserted into an object being examined but also positional fixing with the balloons in the modification 4;
Fig. 18 is a partial side view showing an overtube according to a modification 5;
Fig. 19 explains not only an endoscope, together with an overtube, inserted into an object being examined but also positional fixing with the balloons in the modification 5;
Fig. 20 is a partial side view showing an overtube according to a modification 6;
Fig. 21 explains not only an endoscope, together with an overtube, inserted into an object being examined but also positional fixing with the balloons in the modification 6;
Fig. 22 is a partial side view showing an overtube according to a modification 7;
Fig. 23 explains not only an endoscope, together with an overtube, inserted into an object being examined but also positional fixing with the balloons in the modification 7;
Fig. 24 is an illustration explaining placement of markers in a modification 8;
Fig. 25 explains not only an endoscope, together with an overtube, inserted into an object being examined but also positional fixing with the balloons in the modification 8;
Fig. 26 explains the markers placed for every positional fixing pattern;
Fig. 27 is a perspective view outlining the configuration of a therapeutic system according to a second embodiment of the present invention;
Fig. 28 is a functional block diagram showing electric circuitry in a control box used in the second embodiment;
Fig. 29 is a flowchart outlining how to supply air, which is executed by a CPU incorporated in a controller; and
Fig. 30 is a perspective view outlining a therapeutic system modified from the configuration shown in the second embodiment.

### Detailed Description of Preferred Embodiments

Hereinafter, with reference to the accompanying drawings, various embodiments and their modifications of a therapeutic system that uses an overtube according to the present invention will now be described.

The therapeutic system and treatment method that use the overtube according to the present invention can be applied to not only diagnosis and treatment of body cavities and tubular cavities, such as the usual patients' stomach, duodenal bulb, and duodenum, but also treatment of pancreatic and biliary ducts for the Vater's papilla of a patient who has had a Roux-en-Y reconstructive operation.

### (First embodiment)

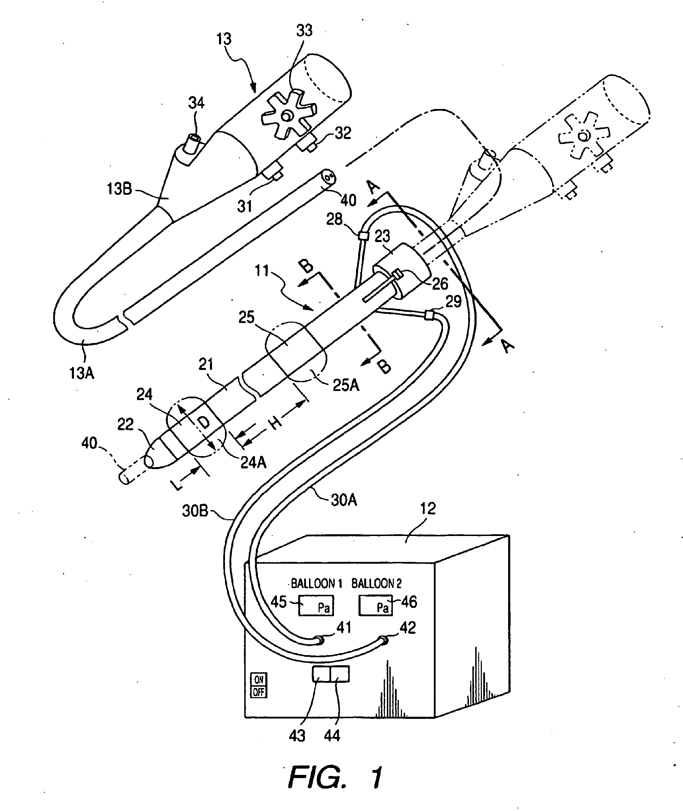

Referring to Figs. 1 - 7, a therapeutic system and a treatment method according to a first embodiment will now be described.

Fig. 1 shows an essential part of the configuration of this therapeutic system. As shown in the figure, the therapeutic system 1 is equipped with an overtube 11 with balloons mounted thereon, an air-supply device 12 that controls the expansion and shrinkage of the balloons on this overtube 11, and an endoscope 13 that is inserted together with the overtube 11 into body cavities and tubular cavities (e.g., orally inserted into the stomach and duodenum) of an object being examined. The endoscope 13 has an insertion tube to be inserted through an insertion channel of the overtube 11, and, with the overtube inserted, the endoscope is inserted into a body cavity or a tubular cavity of the object so as to guide the overtube 11. The overtube 11 is made of resin materials such as thermoplastic elastomer, fluorinated rein, and silicon. This overtube 11 is equipped with a flexible tubular portion 21 which is made into an approximately cylindrical form and is flexible, a distal end portion 22 formed integrally with the distal end of the flexible tubular portion 21, and a grip 23 formed integrally with the other end of the flexible tubular portion 21. In the following, the side which is nearer to the distal end portion 22 will be referred to as the "distal end side," while the side which is nearer to the grip 23 will now be referred to as a "base end side."

The flexible tubular portion 21 is roughly cylindrical, and there is formed an insertion channel P1 into which the insertion tube of the endoscope 13 can be inserted. At mutually different positions in a longitudinal direction (hereinafter called an "axial direction) of the flexible tubular portion 21, a plurality of balloons 24 and 25 are loaded on the outer surface of this tubular portion 21. In the present embodiment, two balloons 24 and 25 are arranged. These balloons 24 and 25 are thin-film flexible pouch members made of resin materials (such as thermoplastic resins including silicon, latex, polyurethane, and nylon) and are loaded in an airtight manner on the outer surface of the flexible tubular portion 21. Thus the balloons 24 and 25 are formed so as to expand or shrink freely by supplying thereto air or discharging air therefrom. It is therefore possible to expand the balloons 24 and 25 within a body cavity or a tubular cavity of an object being examined so as to fix the position of the inserted endoscope 13, i.e., the position of the overtube 11.

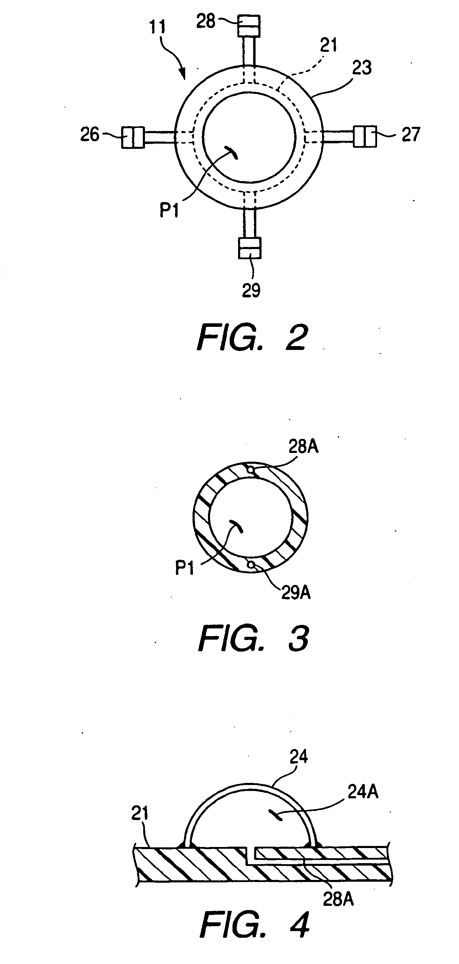

In addition, as shown in Fig. 2, on the flexible tubular portion 21, there are equipped two arm-like water-supply mouth rings 26 and 27, and two arm-like air supply mouth rings 28 and 29. These mouth rings 26 - 29 are formed at four different positions quartering the circumference of the base side end of the flexible tubular portion 21 such that the sleeves protrude from and tilt toward the base end side.

Of these, the water-supply mouth rings 26 and 27 are directly opened to the insertion channel P1 from the inner circumferential surface. Hence when water (such as normal saline) comes from a not-shown water supply source, such as a syringe, the water can moisten the inner circumferential surface.

On the other hand, the air-supply mouth rings 28 and 29 are formed to communicate with air-supply channels 28A and 29A formed through the body of the flexible tubular portion 21 (refer to Fig. 3). The air-supply channels 28A and 29A communicate with inner cavities 24A and 25A of the two balloons 24 and 25, respectively. This structure is exemplified using the balloon 25 in Fig. 4. The air-supply mouth rings 28 and 29 are connected to two air-supply ports 41 and 42 via tubes 30A and 30B, respectively. The air-supply device 12 is provided with two air-supply and air-discharge switches 43 and 44. Using these switches 43 and 44 makes it possible to supply, for example, air through the air-supply ports 41 and 42, independently of each other. When the air is supplied from the air-supply device 12, the air is fed to the air-supply mouth ring 28 (29) via the tube 30A (30B), and then to the balloon 24 (25) via the air-supply channel 28A (29A), thus making the balloon 24 (25) expand. This expanded state of the balloon is pictorially illustrated by a dashed-two dotted line in Fig. 1. Further, the air-supply device 12 is provided with pressure display monitors 45 and 46 that display the inner pressures of the respective balloons 24 and 25.

Each of the balloons 24 and 25 has an inner cubic cavity which is set such that, when each of the balloons 24 and 25 is given a specified amount of air which is decided for every balloon, each balloon is able to expand up to a predetermined size. The inner cubic cavity is set to an amount in a predetermined range that is able to absorb personal differences concerning diameters of target regions (body cavities and tubular cavities) at which the fixing is done using the balloons.

On the other hand, using the switches 43 and 44 to discharge the air allows the air in the balloons 24 and 25 respectively to flow out along the reverse path to the above. Hence the balloons 24 and/or 25 can be shrunk. This shrunk state is pictorially depicted by a solid line in Fig. 1. In this shrunk state, the balloons 24 and 25 are almost flat against the outer surface of the flexible tubular portion 21.

In the present embodiment, the balloons are produced with their geometries and dimensions such that, when a predetermined amount of air is supplied to each of the balloons, the first balloon 24, which is on the distal end side, has an expanded size almost equal to the inner diameter of the duodenum and the second balloon 25, which is next to the first balloon, has an expanded size almost equal to the inner diameter of the duodenal bulb. The geometries and dimensions are chosen respectively depending on the diameters D (each including the diameter of the flexible tubular portion 21) and the lengths L in the axial direction. In addition, a distance H between the balloons 24 and 25 is also decided depending on the length between positions to be fixed (for example, a desired position along the duodenum and the duodenal bulb).

The endoscope 13 is equipped with a flexible, thin and long insertion tube 13A and an at-hand operation device 13B formed integrally with the base-side end of this insertion tube 13A. The at-hand operation device 13B comprises various types of switches 31 and 32 for air-supply/water-supply and suction, as well as an operation lever 33 to bend the insertion tube 13A and a forceps cap 34 to insert a therapeutic forceps (not shown) into the insertion tube 13A. Of these devices, the position of the operation lever 43 is locked by a further lever (not shown) arranged at the at-hand operation device 13B. The insertion tube 13A has a distal end of a predetermined axial length, which is rigid. This distal end portion 40 has a CCD camera and a light source (which are not shown) incorporated therein.

A treatment method executed with the therapeutic system according to the present embodiment will now be exemplified.

First an operator inserts the insertion tube 13A of the endoscope 13 into the insertion channel P1 of the overtube 11. At this time, the balloons 24 and 25 are shrunk. Incidentally, before the insertion, water is supplied via the water-supply mouth rings 26 and 27 of the overtube 11 or from a water-supply channel (not shown) formed though the distal end of the insertion tube 13A of the endoscope, so that the wall of the insertion channel P1 becomes moist. Hence the insertion performance through the insertion channel P1 of the insertion tube 13A is raised. After this moistening, the insertion tube 13A is inserted through the insertion channel P1 such that the distal end portion 40 of the insertion tube 13A slightly protrudes from the distal end portion 22 of the overtube 11 (refer to the dashed-two dotted line in Fig. 1). This secures the field of view for the CCD camera embedded in the distal end portion of the insertion tube 13A.

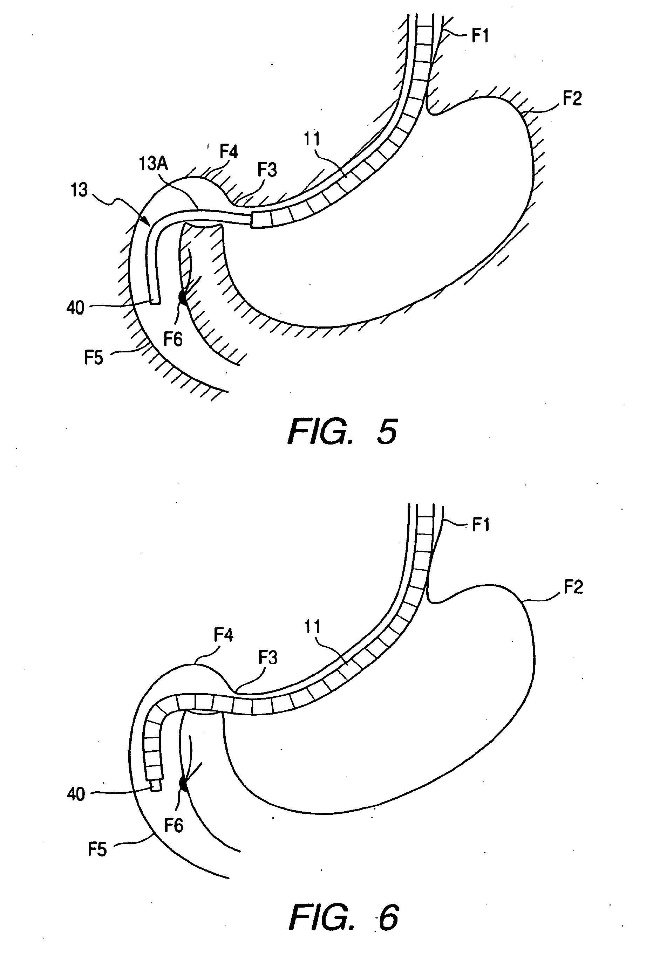

With this insertion state kept, as shown in Fig. 5, for viewing endoscopic images (forward-viewing, side-viewing or oblique-viewing endoscopic images) from the endoscope 13, the operator inserts orally the combined overtube 11 and the insertion tube 13A of the endoscope 13 into the esophagus F1 to pass the stomach F2, pylorus F3, duodenal bulb F4, and duodenum F5 in sequence. And, as shown in Fig. 5, when the distal end portion 40 of the insertion tube 13A reaches a potion near to the Vater's papilla F6 which is located in the duodenum F5, the insertion is stopped.

Then the overtube 11 is manually inserted along the insertion tube 13A of the endoscope 13 to a predetermined position of the distal end portion 40 of the insertion tube 13A, in which the predestined position faces the Vater's papilla F6. This state is shown in Fig. 6.

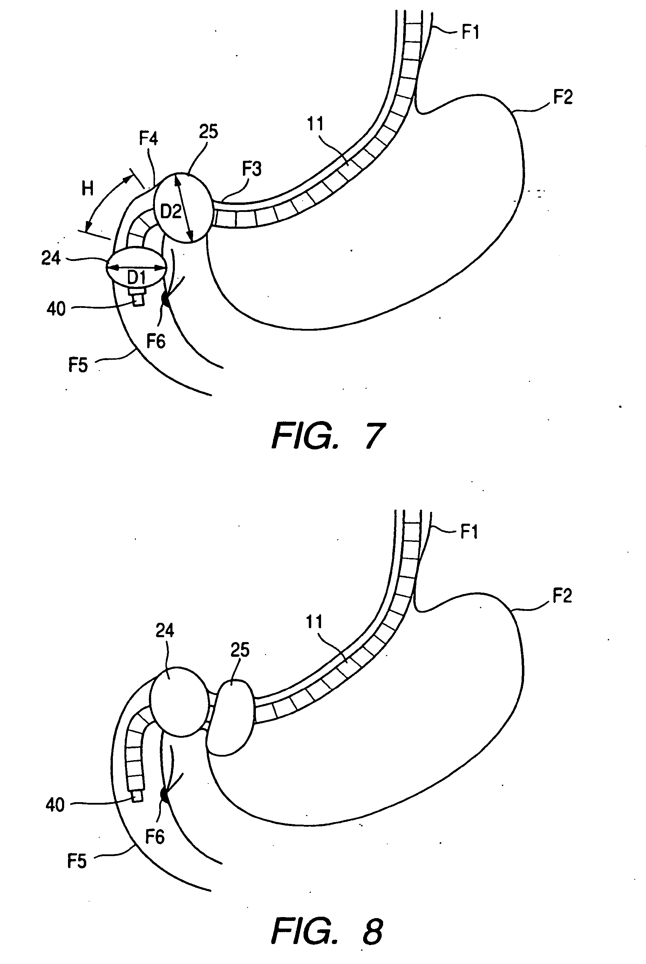

Then the switches 43 and 44 on the air-supply devices 12 are used sequentially to feed a predetermined amount of air to each of the balloons 24 and 25. Responsively to this, the balloons 24 and 25 are expanded in sequence, resulting in one of the two balloons, 24, forcibly coming into contact with the wall of the duodenum F5 and the remaining balloon 25 forcibly coming into contact with the wall of the duodenal bulb F4. This expansion of the balloons takes place under X-ray fluoroscopy to locate and adjust each balloon to the desired position by using fluoroscopic images. Thus, as shown in Fig. 7, the overtube 11 is positionally fixed at the two positions in the duodenum F5 and the duodenal bulb F4. In consequence, the peristaltic motions of a body cavity and any operator's accidental operations cannot shift the position of the overtube 11. Thus the insertion tube 13A of the endoscope 13 can always be located at the position facing the Vater's papilla F6, whereby the endoscope can be used to diagnose and treat the Vater's papilla F6 reliably.

In the present embodiment, the two balloons 24 and 25 are set such that their expanded diameters D1 and D2 which are consistent with the internal diameters of the duodenum F5 and duodenal bulb F4, respectively, and the distance H between the balloons 24 and 25 is substantially equal to the distance between the predetermined position along the duodenum F5 and the duodenal bulb F4. Hence as shown in Fig. 7, expanding both balloons 24 and 25 makes it possible to reliably fix the position of the overtube 11. This facilitates diagnosis and treatment using the endoscope 13.

Incidentally, the number of balloons loaded on the overtube 11 is not necessarily limited to two, but may be three or more. Such a larger number of balloons enable the overtube to be fixed positionally by a larger number of balloons along a body cavity or a tubular cavity, preventing or reducing movement of a body cavity or a tubular cavity from moving the endoscope 13.

### (Modification 1)

Fig. 8 shows a modification 1 which is modified from the foregoing embodiment. This modification 1 is similar to the foregoing embodiment in that the two balloons 24 and 25 are loaded on the overtube 11, except for the positions of the balloons. Practically, both balloons 24 and 25 are located closer to each other in the axial direction, in which one of the balloons, i.e., balloon 24, which precedes the following one, is located at the duodenal bulb F4, while the remaining rear-side balloon 25 is located at the posterior wall of the stomach F2. This makes it possible that, when being expanded, as shown in Fig. 8, the balloons 24 and 25 are located with the pylorus F3 located therebetween for secure positional fixing of the overtube 11 before and after the pylorus F3.

### (Modification 2)

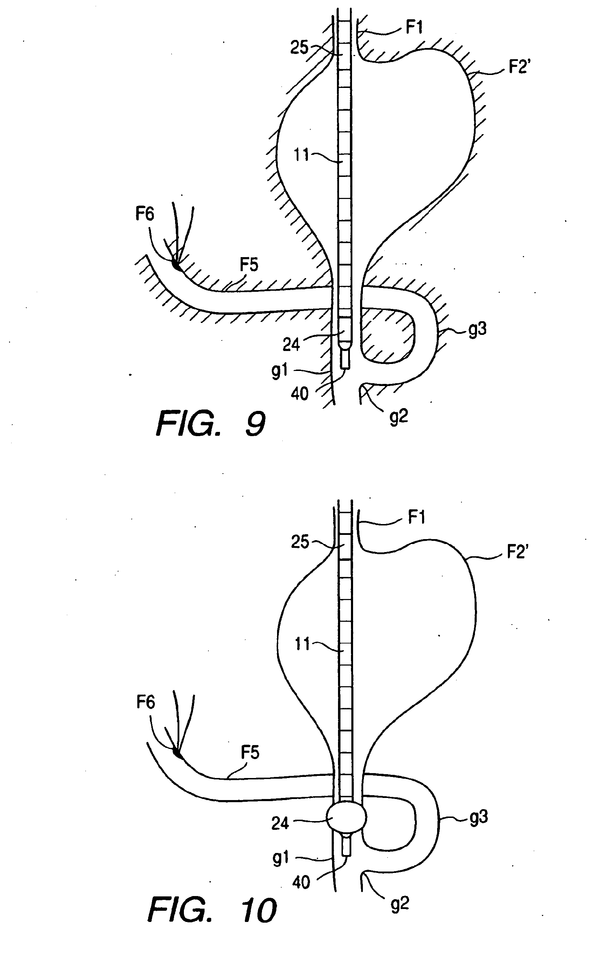

Figs. 9 - 14 show a modification 2 modified from the foregoing embodiment. Like the modification 1, this modification 2 is also similar to the foregoing embodiment in that the two balloons 24 and 25 are loaded on the overtube 11, except for the positions of the balloons. Practically, the overtube 11 according to the second modification 2 is reduced into practice for treatment of pancreatic and biliary ducts to the Vater's papilla of a patient who had the Roux-en-Y gastrectomy.

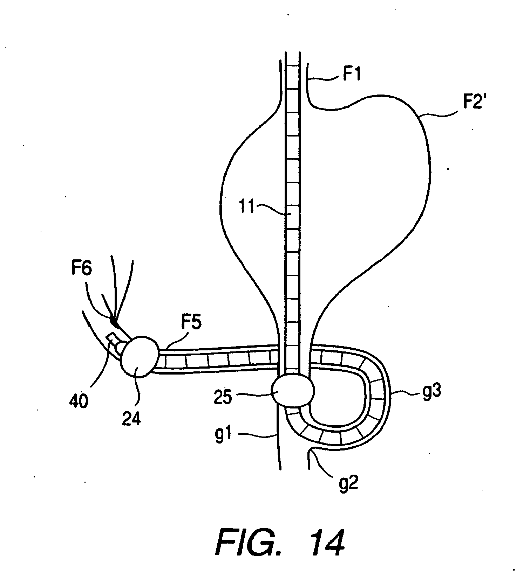

In the modification 2, the distance between both balloons 24 and 25 is longer relative to that shown in the first embodiment and the modification 1 (refer to Fig. 14).

First of all, as shown in Fig. 9, the overtube 11, through which the endoscope 13 is already inserted, is orally inserted into the jejunum g1 via the reconstructed stomach F2', and then temporarily stops the distal end portion 40 of the insertion tube 13A of the endoscope 13 at a given position facing the jejunum-to-jejunum anastomosed potion g2.

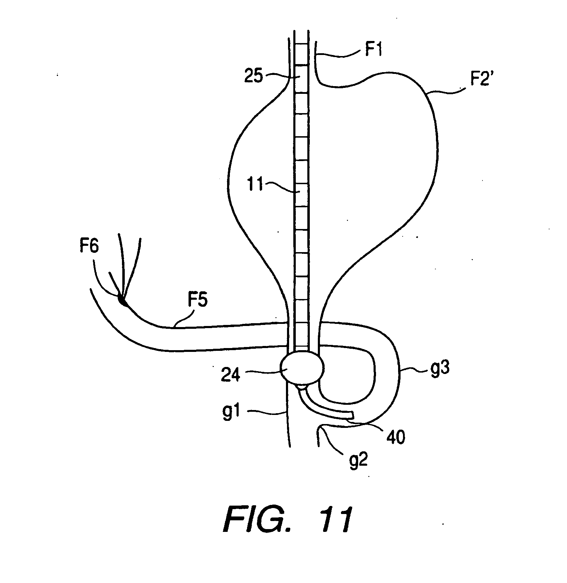

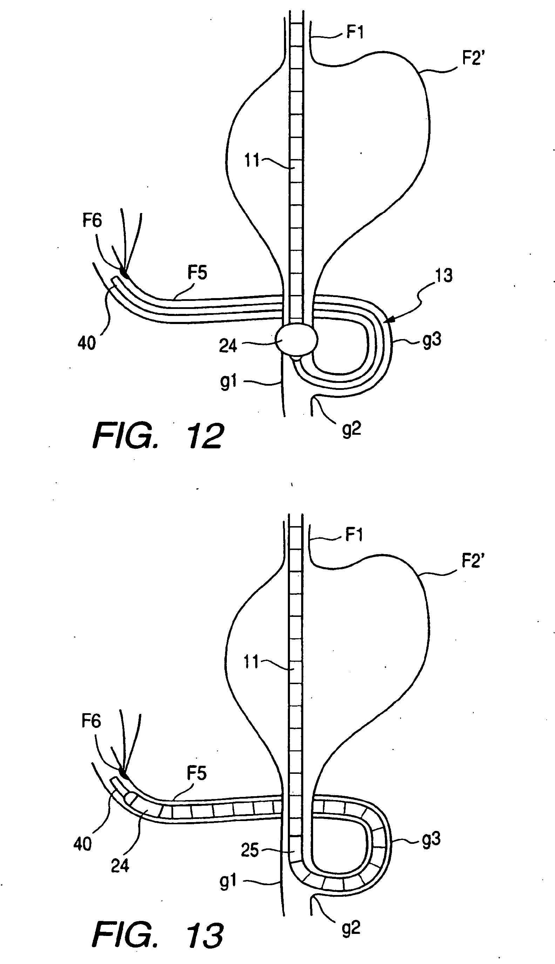

Then, as shown in Fig. 10, the distal-end-side balloon 24 is expanded to temporarily fix the position of the distal end of the overtube 11 at the given position within the jejunum g1, but just before the jejunum-to-jejunum anastomosed portion g2, to the jejunum g1. As shown in Fig. 11, the doctor such as a surgeon operates the endoscope 13 to bend the distal end portion 40 of its insertion tube 13A, during which the endoscope 13 is made to advance further so as to insert the insertion tube 40 into the jejunum-to-jejunum anastomosed portion g2. In this inserted state, the distal end of the overtube 11 is already positionally fixed relative to the jejunum g1, making the insertion operation easier. After this, as shown in Fig. 12, the insertion tube 13A of the endoscope 13 is made to advance further to pass both the jejunum-anastomosed bent portion g3 and the duodenum F5, and arrives at a given position at which the distal end portion 40 of the insertion tube 13A faces the Vater's papilla F6.

The balloon 24, which has been expanded so far, is then shrunk, and the overtube 11 is made to advance along the endoscope 13 so as to reach the position facing the Vater's papilla F6, as illustrated in Fig. 13. In this configuration, the distance between both balloons 24 and 25 is adjusted such that the second balloon 25, which is located on the at-hand side, reaches the position where the distal-end-side lead-off balloon 24 had been present earlier. Using this distance adjustment, the lead-off balloon 24 is again expanded to positionally fix the distal end portion of the overtube 11 to the duodenum F5 at the position just before the Vater's papilla F6. Following this expansion, the rear-side balloon 25 is expanded at a position just before the jejunum-to-jejunum anastomosed portion g2 so as to establish the positional fixing of the overtube 11 relative to the jejunum g1. This situation is illustrated in Fig. 14.

As a result, the position of the overtube 11 is fixed reliably at two positions which serve as key positions in the positional fixing. The endoscope 13 can therefore be used to treat the pancreatic and biliary ducts in a reliable and steady manner. In this way, the approach to the Vater's papilla of a Roux-en-Y gastrectomy patient can be facilitated, contributing to faster and less labor-intensive treatment.

### (Modification 3)

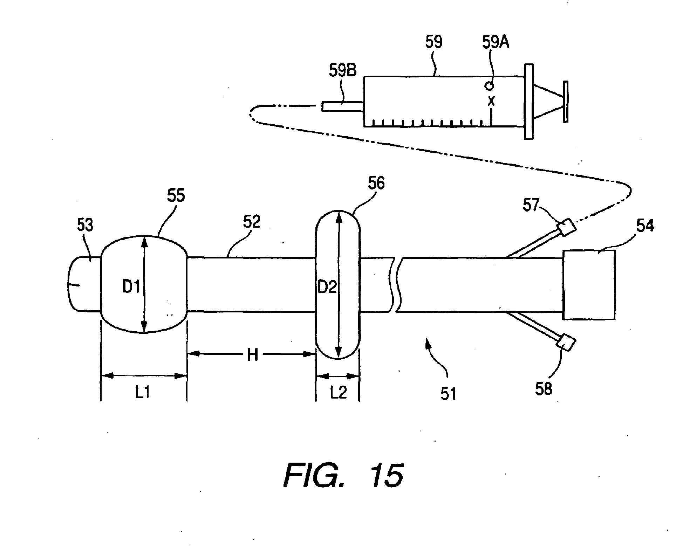

Fig. 15 shows a modification 3 according to the foregoing embodiment. This modification 3 is concerned with an overtube with a simplified air-supply structure.

An overtube 51 shown in Fig. 15 is equipped with a flexible tubular portion 52, and a distal end portion 53 and a grip 54 all formed integrally with both ends of the flexible tubular portion 52. The flexible tubular portion 52 is provided with first and second balloons 55 and 56 located thereon in this arrangement order from the distal end side thereof. The balloons 55 and 56 can be expanded and shrunk. At the flexible tubular portion 52, air-supply mouth rings 57 and 58 are attached for supplying air to the balloons 55 and 56. These structures are identical or similar to those in the first embodiment.

In this modification, the first and second balloons 55 and 56 are different in their axial lengths L from each other, thus thereby providing mutually different diameters D1 and D2 to these balloons when being charged with a predetermined amount of air. For instance, when the balloon length and balloon diameter of the first baboon 55 are denoted as L1 and D1, respectively, and the balloon length and balloon diameter of the second balloon 56 are denoted as L2 and D2, respectively, a relationship of D2>D1 is established, provided that L1>L2 is met. In addition, both balloons 55 and 56 are separated from each other by an axial length H, which corresponds to the distance between the balloon positions when fixed inside body cavity.

Incidentally, the balloon lengths L1 and L2 and amounts of air to be charged are set to the respective specified amounts such that the balloon diameters D1 and D2 are the most appropriate values when being fixed to their different respective target organs.

As air-supply devices that transmit air to the first and second balloon 55 and 56, syringes 59 which have the same capacity are used. The number of syringes 59 may be either two, used separately for the respective balloons 55 and 56, or one, used for both balloons 55 an 56. As shown in Fig. 15, this syringe 59 has a scale thereon, and there is a hole 59A at a position on the scale, the position of which indicates a specified amount of volume. This hole 59A allows the syringe 59 to be charged with only the specified amount of air.

Accordingly, when the first and second balloons 55 and 56 are expanded, the distal end portion 59B of the syringe 59 is inserted into the air-supply mouth rings 57 and 58, before the piston thereof is pushed one time. By this operation, the specified amount of air is supplied to the balloons 55 and 56. In contrast, in cases where each of the first and second balloons 55 and 56 needs to shrink, this syringe 59 is also used for draining the air therefrom.

In this way, a simple-structure and simple-operation syringe 59 can be used to always supply the same amount of air to the first and second balloons 55 and 56 for expanding those balloons. This avoids erroneously supplying an excessive amount of air. It is therefore possible to expand each balloon to have an optimum diameter in a simple manner, in addition to gaining the same or similar advantages gained in the first embodiment. In other words, it is unnecessary to confirm or learn an amount of air being charged into each balloon, simplifying operations for supplying air. Further, there is a secondary merit that erroneous operations can be avoided.

### (Modification 4)

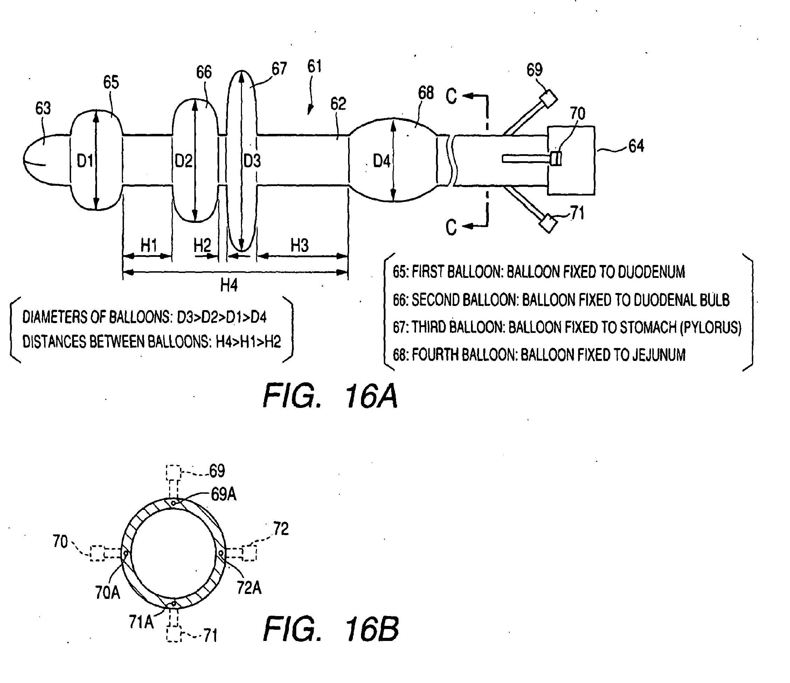

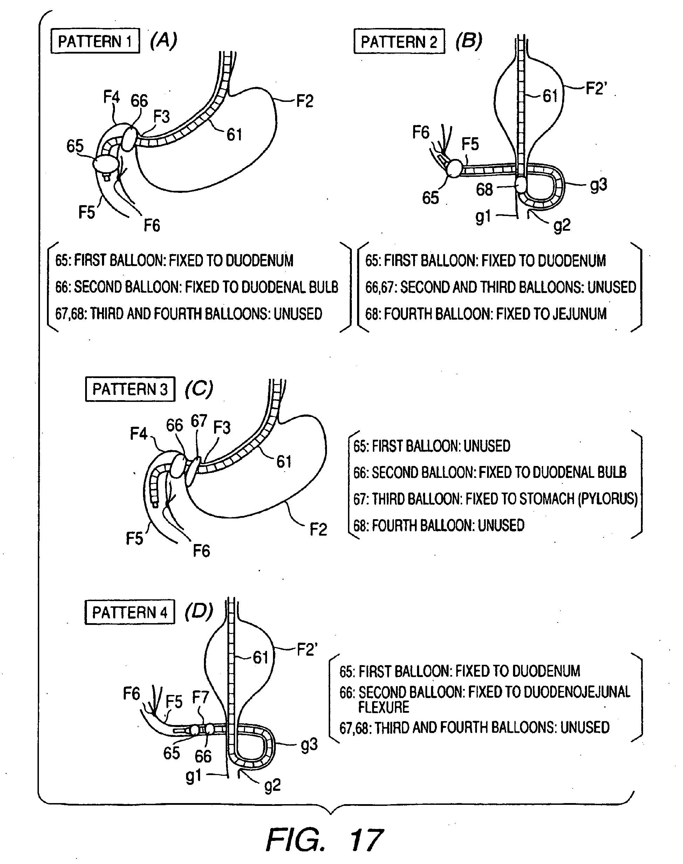

Referring to Figs. 16A, 16B and 17, a modification 4 will now be described.

This modification 4 is developed from the foregoing modification 3. As shown in Fig. 16A, an overtube 61 is equipped with a flexible tubular portion 62, a distal end portion 63 and a grip 64 all formed integrally with both ends of the flexible tubular portion 62. The flexible tubular portion 62 has first to fourth four balloons 65 - 68, which can be expanded and shrunk and are arranged in this order from the distal end thereof. On the flexible tubular portion 62, air-supply mouth rings 69 - 72 are loaded to provide air to the balloons 65 - 68 respectively (for the pile sleeve 72, refer to Fig. 16B). The air-supply mouth rings 69 - 72 are produced to communicate with the balloons 65 - 68, respectively, via each of air-supply channels 69A - 72A. These components themselves are similar or identical in structures to those in the first embodiment.

The first to forth balloons 65 - 68 are decided in advance concerning which target portion (i.e., an inner portion of a body cavity of a tubular cavity) each balloon is to be fixed to. Depending on the type of each target portion, the diameter D of each balloon, length L of each balloon, and an axial distance H between balloons are decided previously. In addition, in the present embodiment, a pattern (in the following, called a positional fixing pattern) in which the balloons (i.e., the overtube 61) are positionally fixed to respective target portions is classified into four types of patterns (patterns 1 - 4 shown in Fig. 17).

At first, the geometries of the first to fourth balloons 65 - 68, which are expanded by supplying the same specified amount (i.e., maximum amount) of air to each balloon, will now be described. For supplying the same specified amount of air, the present example can employ the foregoing syringe 59.

As to the diameters of the balloons, the axial length of each balloon is set in advance such that
- the first balloon 65 is a balloon fixed to the duodenum and has a desired amount of balloon diameter D1 falling into an allowable range of 20 - 50 mm when being expanded by a specified amount of air,
- the second balloon 66 is a balloon fixed to the duodenal bulb and has a desired amount of balloon diameter D2 falling into an allowance of 20 - 60 mm when being expanded by supply of the specified amount of air,
- the third balloon 67 is a balloon fixed to the stomach (pylorus) and has a desired amount of balloon diameter D3 falling into an allowance of 30 - 70 mm when being expanded by supply of the specified amount of air, and
- the fourth balloon 68 is a balloon fixed to the jejunum and has a desired amount of balloon diameter D4 falling into an allowance of 20 - 40 mm when being expanded by supply of the specified amount of air, respectively. The diameter D2 may be equal to the diameter D1. Thus, when comparison is made among the maxima of the respective allowances, a relationship of D3>D2≥D1>D4 is realized.

On the other hand, the axial distances between balloons are set previously such that
- a distance H1 between the first and second balloons 65 and 66 is a desired amount falling into an allowance of 30 - 100 mm,
- a distance H2 between the second and third balloons 66 and 67 is a desired amount falling into an allowance of 5 - 20 mm,
- a distance H4 between the first and fourth balloons 65 and 68 is a desired amount falling into an allowance of 200 - 500 mm (however, including the balloon lengths of the second and third balloons 66 and 67), and
- a distance H3 between the third and fourth balloons 67 and 68 is an amount decided in accordance with the distances H1, H2 and H4. Thus, as to the distances between balloons, a relationship of H4>H1>H2 is realized.

The reason why the allowances are prepared for the diameters and the distances as above is to take the individual differences of patients into account. Therefore, for practical use, a plurality of types of overtubes 61 are prepared beforehand which have combinations of different balloon diameters, and a plurality of types of overtubes 61 are also prepared beforehand which have combinations of different distances between balloons. Alternatively, when an amount of air to be injected is set to a specified amount 11, an overtube with balloons which can be expanded up to a maximum value for each balloon may be prepared, and, if required, the amount of injected air may be set to a specified amount 12 (for all the balloons) less than the specified amount I1.

Depending on the requirements, these first to fourth balloons 65 - 68 are targeted on part of a body cavity or a tubular cavity for positional fixing. Four example types of positional fixing patterns are given here.
- the first positional fixing pattern (pattern 1) is a pattern where, as shown in Fig. 17(A), the first balloon 65 is fixed to the duodenum F5, the second balloon 66 is fixed to the duodenal bulb F4, and the remaining third and fourth balloons 67 and 68 are unused (not expanded),
- the second positional fixing pattern (pattern 2) is applied to treatment of the pancreatic and biliary ducts of the Vater's papilla of a Roux-en-Y gastrectomy patient, where as shown in Fig. 17(B), the first balloon 65 is fixed to the duodenum F5, the second and third balloons 66 and 67 are unused, and the fourth balloon 68 is fixed to the jejunum g1,
- the third positional fixing pattern (pattern 3) is a pattern where, as shown in Fig. 17(C), the first balloon 65 is unused, the second balloon 66 is fixed to the duodenal bulb F4, the third balloon 67 is fixed to the stomach (pylorus) F3, and the fourth balloon 68 is unused (not expanded), and
- the fourth positional fixing pattern (pattern 4) is applied to treatment of the pancreatic and biliary ducts of the Vater's papilla of a Roux-en-Y gastrectomy patient, where as shown in Fig. 17(D), the first balloon 65 is fixed to the duodenum F5, the second balloon 66 is fixed to the duodenojejunal flexture F7, and the remaining third and fourth balloons 67 and 68 are unused (not expanded).

As a result, a doctor including a surgeon is able to select a proper overtube 61 according to the route along which an approach is made towards a target portion for diagnosis and treatment and also according to individual differences in sizes of body cavities and tubular cavities. When the selected overtube 61 is inserted, the air is supplied only to balloons to be used, on the basis of a desired positional fixing pattern. This provides reliable positional fixing for any of the first to fourth positional fixing patterns. In particular, a single overtube can be used for various types of positional fixing which are based on the first to the fourth patterns. Thus, it is sufficient that the types of overtubes take only individual differences into account. It is not necessary to increase the number of overtubes according to the number of portions to be treated. It is therefore possible to reduce the types of overtubes kept in stock. In addition, the overtube according to the present modification can be used for treatment of the pancreatic and biliary ducts of a patient who has had a Roux-en-Y reconstructive operation or a normal endoscopic retrograde cholangiopancreatography (ERCP), providing versatility of application.

Incidentally, the number of balloons is not always limited to the foregoing first to fourth four balloons 65 - 68, but may be at least two of the first to fourth balloons 65 - 68, which are combined as desired.

### (Modification 5)

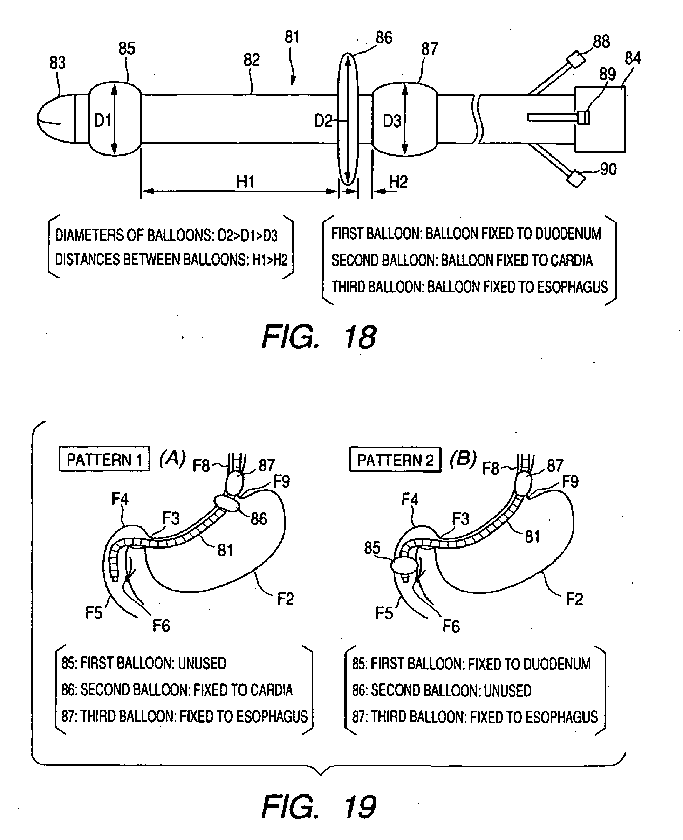

Referring to Figs. 18 and 19, a modification 5 will now be described.

This modification 5 is developed from the foregoing modification 3, like the modification 4. As shown in Fig. 18, an overtube 81 is equipped with a flexible tubular portion 82, and a distal end portion 83 and a grip 84 both formed integrally with both ends of the flexible tubular portion 82. The flexible tubular portion 82 has first to third three balloons 85 - 87, which can be expanded and shrunk and are arranged in this order from the distal end thereof. On the flexible tubular portion 82, air-supply mouth rings 88 - 90 are loaded to provide air to the balloons 85 - 87. The air-supply mouth rings 88 - 90 are produced to communicate with the balloons 85 - 87, respectively, via not-shown air supply channels. These components themselves are similar or identical in structure to those in the first embodiment.

Like the foregoing modification 4, the first to third balloons 85 - 87 are targeted in advance depending on which target portion each balloon is to be fixed to. Depending on the type of each target portion, the diameter D of each balloon and an axial distance H between balloons are set previously. In addition, in the present embodiment, the positional fixing for those balloons is given in two example patterns.

First, the geometries of the first to third balloons 85 - 87, which are expanded by supplying the same specified amount (i.e., maximum amount) of air to each balloon, will now be described. For supplying the same specified amount of air, the present example may employ the foregoing syringe 59.

Regarding the diameters of the balloons,
- the first balloon 85 is a balloon fixed to the duodenum F5 and has a desired amount of balloon diameter D1 falling into an allowance of 20 - 50 mm when being expanded by supply of a specified amount of air,
- the second balloon 86 is a balloon fixed to the cardia F9 and has a desired amount of balloon diameter D2 falling into an allowance of 30 - 70 mm when being expanded by supply of a specified amount of air, and
- the third balloon 87 is a balloon fixed to the esophagus F8 and has a desired amount of balloon diameter D3 falling into an allowance of 15 - 25 mm when being expanded by supply of a specified amount of air, respectively. Thus, when comparison is made among the maxima of the respective allowances, a relationship of D2>D1>D3 is realized.

On the other hand, the axial distances between balloons are set such that
- a distance H1 between the first and second balloons 85 and 86 is a desired amount falling into an allowance of 150 - 300 mm, and
- a distance H2 between the first and second balloons 86 and 87 is a desired amount falling into an allowance of 5 - 20 mm. Thus, as to the distances between balloons, a relationship of H1>H2 is realized.

In this case, the reason why the allowances are prepared for the diameters and the distances as above is for taking individual differences of patients into account.

Depending on needs for which, of these first to third balloons 85 - 87, which balloon should be used at which part of a body cavity or a tubular cavity for positional fixing, two types of positional fixing patterns are prepared. Practically,
- the first positional fixing pattern (pattern 1) is a pattern where, as shown in Fig. 19(A), the first balloon 85 is unused, the second balloon 86 is fixed to the cardia F9 and the remaining third balloon 87 is fixed to the esophagus F8, and
- the second positional fixing pattern (pattern 2) is a pattern where, as shown in Fig. 19(B), the first balloon 85 is fixed to the duodenum F5, the second balloon 86 is unused, and the third balloon 87 is fixed to the esophagus F8.

As a result, doctors including surgeons are able to select the correct overtube 81 for patients who have not had a Roux-en-Y reconstructive operation, taking into account individual differences in sizes of internal organs and portions being targeted for diagnosis and treatment. When the selected overtube 81 is inserted, air is supplied only to balloons to be used, on the basis of the desired positional fixing pattern. This provides the single overtube 81 with a reliable positional fixing function on any of the first to second positional fixing patterns. In consequence, for performing the ERCP with patients, identical or similar advantages to those in the modification 4 can still be obtained.

### (Modification 6)

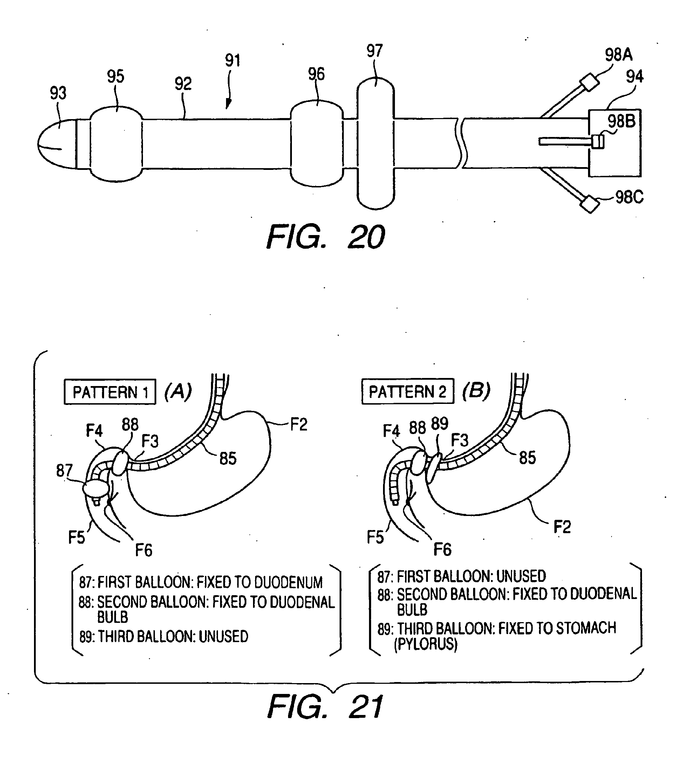

Referring to Figs. 20 and 21, a modification 6 will now be described. This modification 6 is concerned with an overtube in which the internal cavity volume is changed depending on the type of portion being subjected to positional fixing.

An overtube 91 shown in Fig. 20 is equipped with a flexible tubular portion 92, and a distal end portion 93 and a grip 94 both formed integrally with both ends of the flexible tubular portion 92. The flexible tubular portion 92 has first to third three balloons 95, 96 and 97, which can be expanded and shrunk and are arranged in this order from the distal end thereof. On the flexible tubular portion 92, air-supply mouth rings 98A - 98C are loaded to provide air to the balloons 95 - 97. These components are similar or identical in structures to those in the first embodiment.

The first balloon 95 is loaded for being fixed to the duodenum F5, the second balloon 96 is loaded for being fixed to the duodenal bulb F4, and the third balloon 97 is loaded for being fixed to the stomach (pylorus) F3, respectively.

The axial distances between balloons among the first to third balloons 95, 96 and 97 and the diameters expanded in a direction to the axial direction are set in the following manner. For the balloon-to-balloon axial distances, a relationship of "the distance between the second balloon 96 (to be fixed to the duodenal bulb) and the third balloon 97 (to be fixed to the stomach)" < "the distance between the first balloon 95 (to be fixed to the duodenal bulb) and the second balloon 96" is maintained. As for the diameters, the first balloon 95 (to be fixed to the duodenum), the second balloon 96 (to be fixed to the duodenal bulb), and the third balloon 97 (to be fixed to the stomach) have desired amounts, respectively, which are successively larger in this order.

Depending on how the first to third balloons 95, 96 and 97 are to be used for positional fixing (i.e., which balloon(s) are expanded), two types of positional fixing patterns are described here.

The first positional fixing pattern (pattern 1) is used such that, as shown in Fig. 21(A), the first balloon 95 is fixed to the duodenum F5, the second balloon 96 is fixed to the duodenal bulb F4, and the third balloon 97 is unused (not expanded). The second positional fixing pattern (pattern 2) is used such that, as shown in Fig. 21(B), the first balloon 95 is unused, the second balloon 96 is fixed to the duodenal bulb F4, and the third balloon 97 is fixed to the stomach (pylorus) F3.

Hence, the single overtube 91 alone can be used to provide a steady positional fixing function on the basis of either the first or second positional fixing pattern.

In a further modified form, the third balloon 97 may be used as a common balloon selectively fixed to both the stomach F2 and the jejunum g1. In such a case where the third positional fixing pattern for fixing to the jejunum g1, an amount of air supplied from a not-shown air-supply device to the third balloon 97 is adjusted to be less than an amount required for the balloon 97 to be fixed to the stomach F2. According to this modified form, one balloon is used as a positional fixing balloon at various target portions, thus increasing the types of positional fixing patterns available, thus enhancing versatility of the balloons, i.e., the overtube.

### (Modification 7)

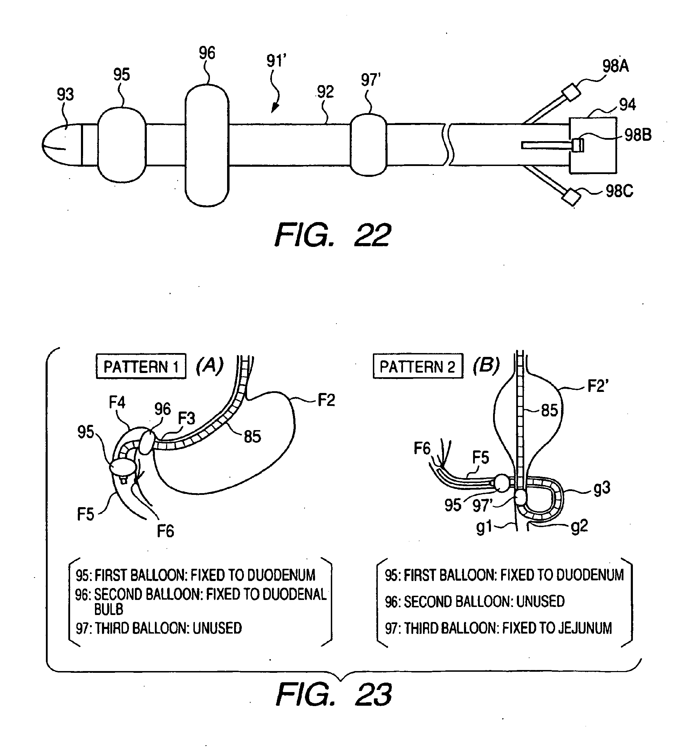

Referring to Figs. 22 and 23, a modification 7 will now be described. This modification 7 is further modified from the foregoing sixth modification 6, with components which are the same or similar as or to the components shown in the modification 6 are given the same reference numerals in the modification 6.

Fig. 22 shows an overtube 91' according to the modification 7. This overtube 91' differs from the overtube 91 according to the modification 6 in the following respects. Although the third balloon 97 in the modification 6 is for fixing to the stomach (pylorus) F3, whereas the third balloon 97' of the overtube 91' according to this modification 7 is loaded for fixing to the jejunum g1. Further, the quantitative relationships of axial distances between the first balloon 95 (fixed to the duodenum), the second balloon 96 (fixed to the duodenal bulb), and the third balloon 97' (fixed to the jejunum) are also different from those of the modification 6, as will be described later. The remaining configurations are the same as those in the modification 6.

The axial distances between the first to third balloons 95, 96 and 97' and the diameters of these balloons expanded in the direction perpendicular to the axial direction are set as follows. Practically, the balloon-to-balloon distances are set to maintain a relationship of "the distance between the first balloon 95 (fixed to the duodenum) and the second balloon 96 (fixed to the duodenal bulb) < the distance between the second balloon 96 and the third balloon 97' (fixed to the jejunum)." Additionally, the diameters are set such that the third balloon 97' (fixed to the jejunum), the first balloon 95 (fixed to the duodenum), and the second balloon 96 (fixed to the duodenal bulb) have desired values, respectively, which become larger in this arrangement order.

In the present modification 7, depending on needs for how the first to third balloons 95, 96 and 97' are used for positional fixing (i.e., expanded), two types of positional fixing patterns are prepared.

Specifically, the first positional fixing pattern (pattern 1) is such that, as shown in Fig. 23(A), the first balloon 95 is for being fixed to the duodenum F5, the second balloon 96 is for being fixed to the duodenal bulb F4, and the third balloon 97' is unused (not expanded). In the second positional fixing pattern (pattern 2), as shown in Fig. 23(B), the first balloon 95 is fixed to the duodenum F5, the second balloon 96 is unused, and the third balloon 97' is fixed to the jejunum g1.

Hence, only the single overtube 91' can be used to provide a steady positional fixing function on the basis of either the first or second positional fixing pattern.

### (Modification 8)

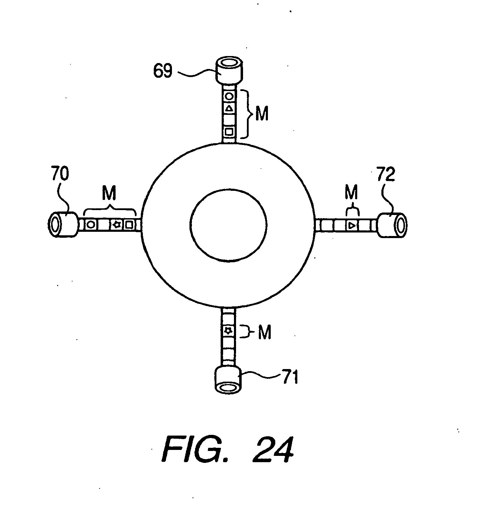

Referring to Figs. 24 - 26, a modification 8 will now be described. This modification 8 relates to a configuration in which markers are put on air-supply ports used for every positional fixing pattern in view of avoiding operator's erroneous operations.

Fig. 24 shows an axial external view of four arm-like air-supply mouth rings 69 - 72 serving as air-supply ports, with these sleeves mounted on the overtube 61 equipped with the four balloons 65 - 68 as shown in Figs. 16A and 16B. On the arm parts of these air-supply ports 69 - 72, there are provided markers M assigned in common to each of the positional fixing patterns. A relationship between the markers M and the positional fixing patterns will now be exemplified as follows.

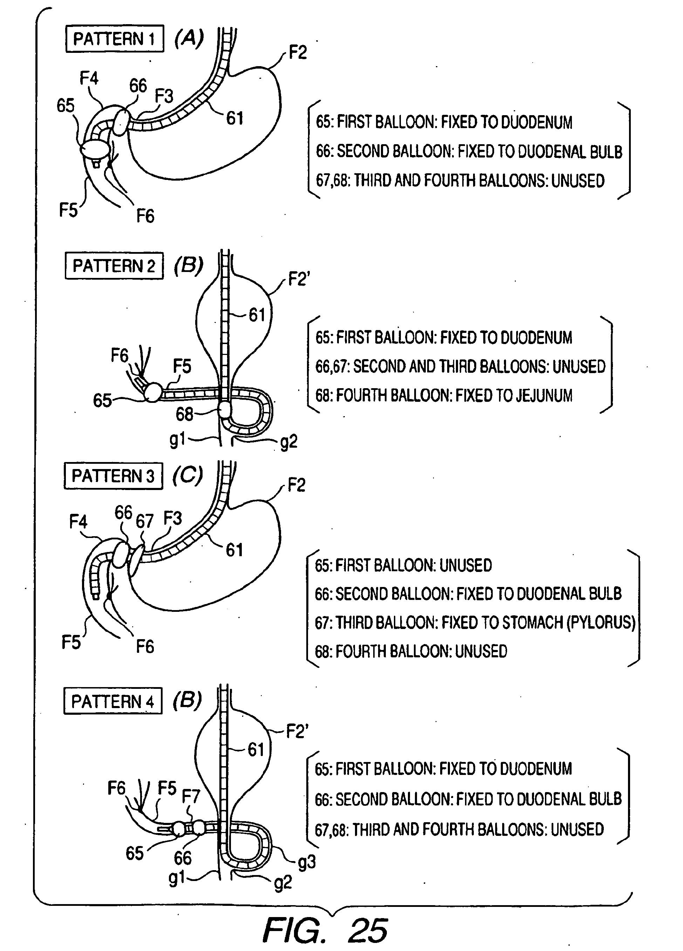

It is now assumed that the positional fixing patterns are:
- the first positional pattern in which, as shown in Fig. 25(A), the first balloon 65 is fixed to the duodenum F5, the second pattern 66 is fixed to the duodenal bulb F4, and the remaining third and fourth balloons 67 and 68 are unused (not expanded);
- the second positional pattern in which, as shown in Fig. 25(B), the first balloon 65 is fixed to the duodenum F5, the second and third balloons 66 and 67 are unused, and the fourth balloon 68 is fixed to the jejunum g1;
- the third positional pattern in which, as shown in Fig. 25(C), the first balloon 65 is unused, the second balloon 66 is fixed to the duodenal bulb F4, the balloon 67 is fixed to the stomach (pylorus) F3, and the fourth balloon 68 is unused; and
- the fourth positional pattern in which, as shown in Fig. 25(D), the first balloon 65 is fixed to the duodenum F5, the second balloon 66 is fixed to the duodenojejunal flexture F7, and the third and fourth balloons 67 and 68 are unused (not expanded).

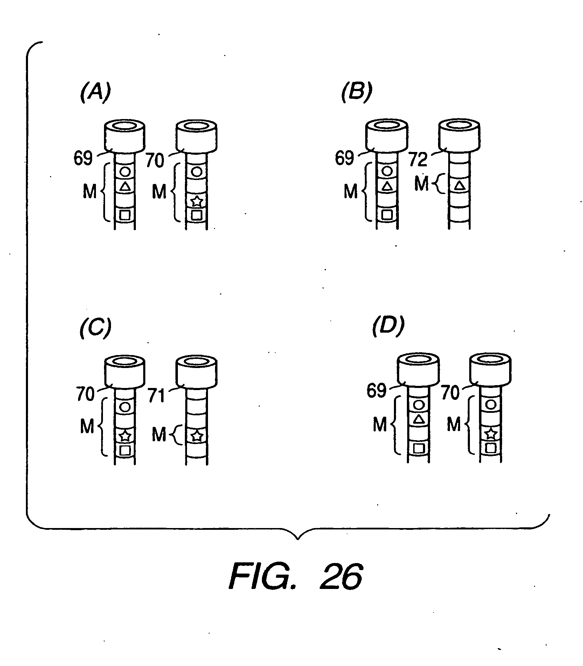

The markers M are placed such that:
- to show the first positional fixing pattern, as shown in Fig. 26(A), a common marker "circle (o)" is put on the arm parts of the first and second air-supply mouth rings 69 and 70 which communicate with the first and second balloons 65 and 66, respectively;
- to show the second positional fixing pattern, as shown in Fig. 26(B), a common marker "triangle (Δ)" is put on the arm parts of the first and fourth air-supply mouth rings 69 and 72 which communicate with the first and fourth balloons 65 and 68, respectively;
- to show the third positional fixing pattern, as shown in Fig. 26(C), a common marker "asterisk (*)" is put on the arm parts of the second and third air-supply mouth rings 70 and 71 which communicate with the second and third balloons 66 and 67, respectively; and
- to show the fourth positional fixing pattern, as shown in Fig. 26(D), a common marker "square (□)" is put on the arm parts of the first and second air-supply mouth rings 69 and 70 which communicate with the first and second balloons 65 and 66, respectively.

In this way, a wide variety of types of markers are put on the arm-parts of the air-supply mouth rings, as a variety of types of patterns are used. The same type of markers that are placed in common on plural arms indicates air-supply ports to be used in the same positional fixing pattern. Accordingly, an operator is given a clear indication showing which combination of air-supply mouth rings should be used when air-supply means, such as syringes, are inserted into or connected to the sleeves. It is thus possible to prevent erroneous operations such as incorrect connection of the air supply.

A modified form from the above configuration is also provided. The positions at which the markers are put are not necessarily limited to the arm parts of the air-supply mouth rings, but may be for example the outer face of the flexible tubular portion, provided that such positions are still near the air-supply mouth rings. Another modified form is that the markers are not limited to only symbols such as circles and triangles, but may be numbers.

### (Second embodiment)

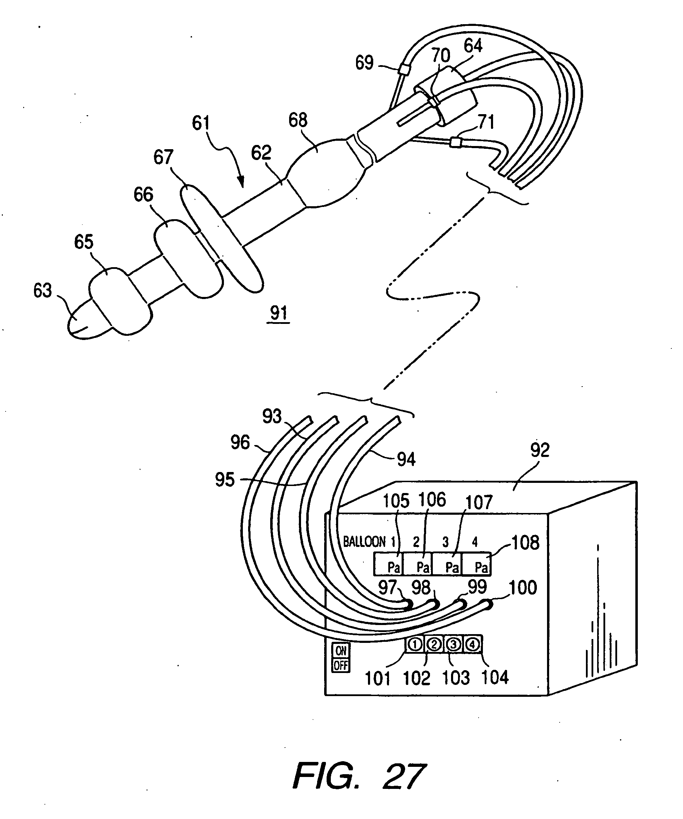

Referring to Figs. 27 - 29 (and Figs. 16A, 16B and 17A- 17D), a second embodiment of the therapeutic system according to the present invention will now be described.

The second embodiment relates to a configuration where the foregoing plural types of positional fixing patterns are preset so that only selecting a desired pattern makes it possible to supply a specified amount of air to each of the balloons based on the selected pattern. In the following, the components similar or identical to those in the foregoing embodiment and modifications are given the same references for the sake of a simplified explanation.

As shown in Fig. 27, a therapeutic system 91 according to the present embodiment is equipped with the overtube 61 having four balloons and a control box for controlling supply and discharge of air to and from the four balloons. Specifically, the overtube 61 is equipped with the first to fourth balloons 65 - 68, and its configuration is totally consistent with that shown in the modification 4. For this reason, the same reference numerals as those in Figs. 16A and 16B are used again.

Of this overtube 61, the mouth rings 69 - 72 respectively communicate with the first to fourth balloons 65 - 68 and are connected with air-supply ports 97, 98, 99 and 100 of the control box via four tubes 93, 94, 95 and 96, respectively.

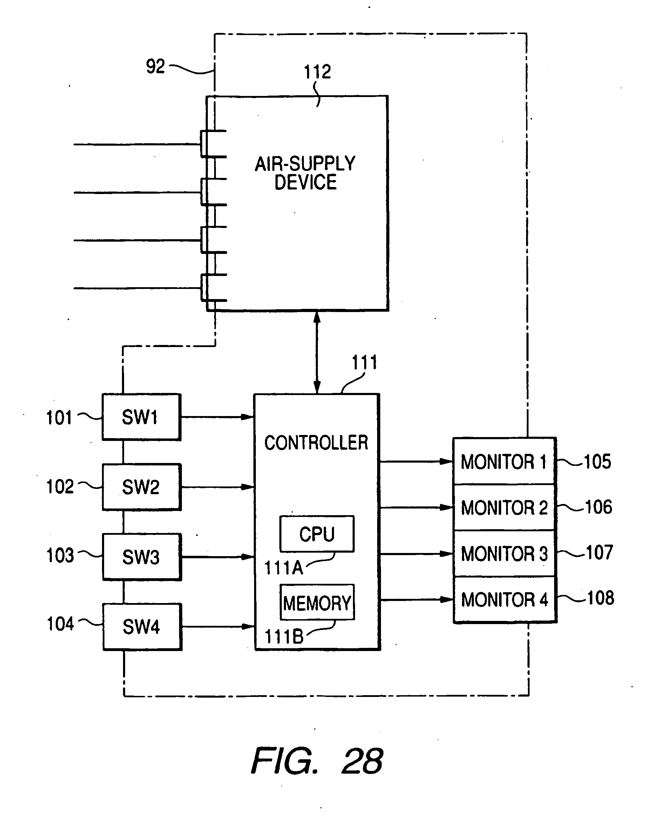

The control box 92 is further detailed in Fig. 28, wherein there are provided a controller 111 equipped with a computer including a CPU 111A and a memory 111B, and an air-supply devices 112 that automatically supplies air from a specified one of the air-supply ports 97 - 100 in response to a control signal coming from the controller 111. The computer executes software processing for the air-supply control. The controller 111 receives output signals from pattern selection switches 101, 102, 103 and 104 which are able to select each of the foregoing positional fixing patterns. The pattern selection switches 101 - 104 are placed on the front of the casing of this control box 92, so that an operator operates the switches by hand.

In the embodiment, operating (switching on) the first pattern selection switch 101 selects the first positional fixing pattern, which is the same as shown in Fig. 17(A). Operating the second pattern selection switch 102 selects the second positional fixing pattern, which is the same as shown in Fig. 17(B). Likewise, operating the third pattern selection switch 103 selects the third positional fixing pattern, which is the same as shown in Fig. 17(C). Furthermore, operating the fourth pattern selection switch 104 selects the fourth positional fixing pattern, which is the same as shown in Fig. 17(D). When the switch 101 (to 104), which was once pressed for the air supply, is pressed again, the air is discharged.

In addition, on the front of the control box 92 are provided pressure display monitors 105, 106, 107 and 108. These monitors 105 - 108 are capable of displaying pressure signals coming from the air-supply device 112. Responding to control signals (including a balloon selection signal) coming from the controller 111, the air-supply device 112 has the capability of supplying a specified amount of air via a specified one of the air-supply ports 98 - 100, which specified air-supply port corresponding to the balloon selection. This device also has the capability of outputting to the monitor 105 (-108) a signal indicating a pressure value in the internal cavity of a balloon selected for the air supply.

In the controller 111, the CPU 111A reads air-supply programs, previously installed in the memory 111B, from the memory111B into a work area thereof, and then sequentially executes procedures coded in the programs for the air supply control.

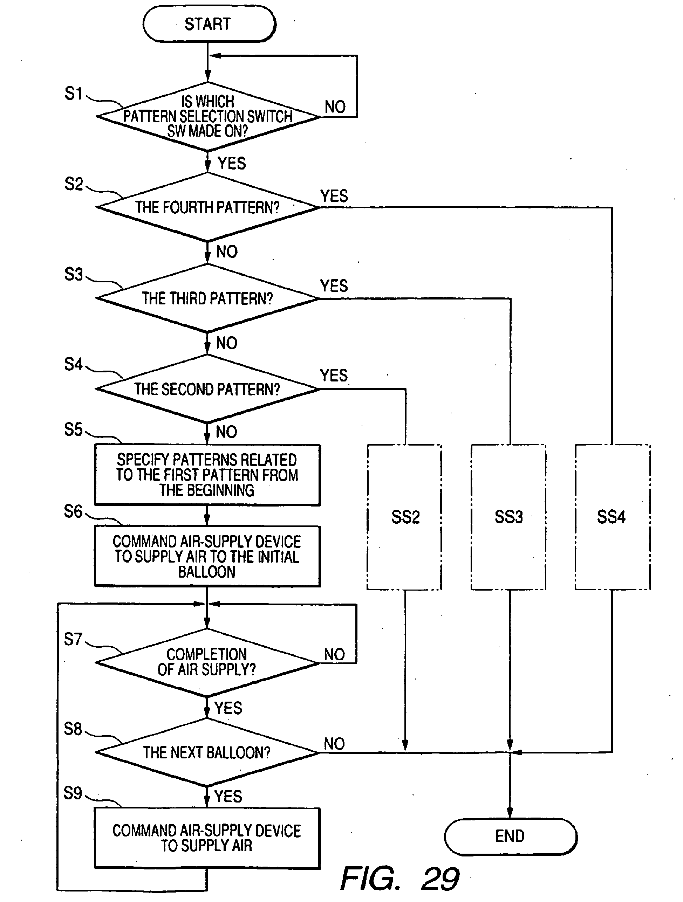

In the present example, the CPU 111A first waits for the output signals from the switches to determine whether an operator operates any of the pattern selection switches 101, 102, 103 and 104 by monitoring the signals in turn (Fig. 29, step S1). When the determination at step S1 exhibits that any switch 101 (- 104) has been pressed (ON), the CPU 111A decides which switch, that is, which pattern, has been selected in a sequential manner (steps S2 - S4). Specifically, the determination of whether or not the fourth pattern selection switch 104 has been operated leads to whether or not the fourth positional fixing pattern is selected (step S2). The determination of whether or not the third pattern selection switch 103 has been operated leads to whether or not the third positional fixing pattern is selected (step S3). The determination whether or not the second pattern selection switch 102 has been operated leads to whether or not the second positional fixing pattern has been selected (step S4). The determination "NO" at any of steps S2, S3 and S4 means that the first positional fixing pattern has been selected.

In response to this selection, the CPU 111A decides which balloons which should be activated (expanded) on the first positional fixing pattern as wall as the alignment sequence of the balloons from the head (step S5). In this case, the balloons to be activated are the first and second balloons 65 and 66, as shown in Fig. 17(A), and the alignment sequence from the head is a sequence along which the first balloon 65 and the second balloon 66 are lined up in this order. As described before, the third and fourth balloons 67 and 68 are not activated.

Thus, to supply air to the balloon located at the forefront of the sequence, that is, the first balloon 65, a control signal is sent to the air-supply device 12 (step S6). By this control, the air-supply device 112 is driven to supply a specified amount of air from the air-supply port 97 to expand the first balloon 65. Since a reply from the air-supply device 112 notifies the CPU 111A of completion of the air supply (YES at step S7), the CPU 111A determines whether or not there is one or more remaining balloons to be activated (step S8). In this pattern, the second balloon 66 remains to be inflated, so that the air-supply device 112 is commanded to supply air to this second balloon 66 (step S9). Thus the air-supply device 112 sends out the air via the air-supply port 98, so that the second balloon 66 can be expanded. The CPU 111A repeats the processes at steps S7 - S9 so as to complete the air supply to all the balloons which should be activated (NO at step S8), before ending the air supply control.

Meanwhile, in the case of the determination YES at step S4, that is, the second positional fixing pattern is selected, similar processing to that in steps S5 - S9 is executed to command the activation (expansion) of all the balloons according to the second positional fixing pattern (step SS2). In this pattern, as shown in Fig. 17(B), the first and fourth balloons 65 and 68 are objective balloons which should be activated, and their alignment sequence from the head becomes a sequence along which the first balloon 65 and the fourth balloon 68 are lined up in this order. The air is supplied to only those balloons 65 and 68.

Moreover, in the case of the determination YES at step S3, that is, the third positional fixing pattern is selected, the similar processing to that in steps S5 - S9 is executed to command the activation (expansion) of all the balloons according to the third positional fixing pattern (step SS3). In this pattern, as shown in Fig. 17(C), the second and third balloons 66 and 67 are objective balloons which should be activated, and their alignment sequence from the head becomes a sequence along which the second balloon 66 and the third balloon 67 are lined up in this order. The air is supplied to only those balloons 66 and 67.

In the case of the determination YES at step S2, that is, the fourth positional fixing pattern is selected, similar processing to that in steps S5 - S9 is executed to command the activation (expansion) of all the balloons according to the fourth positional fixing pattern (step SS4). In this pattern, as shown in Fig. 17(D), like the first positional fixing pattern, the first and second balloons 65 and 66 are objective balloons which should be activated, and their alignment sequence from the head is the second balloon 66 and then the third balloon 67 which are lined up in this order. The air is supplied to only those balloons 65 and 66.

Accordingly, the present embodiment provides various preset combinations of plural balloons, so that simply selecting a desired preset combination allows the necessary plural balloons to be expanded in sequence from the balloon located at the forefront of the sequence, making the positional fixing easier. Identical or similar advantages to those gained in the first embodiment can also be obtained, reducing operator work load significantly.

### (Modification)

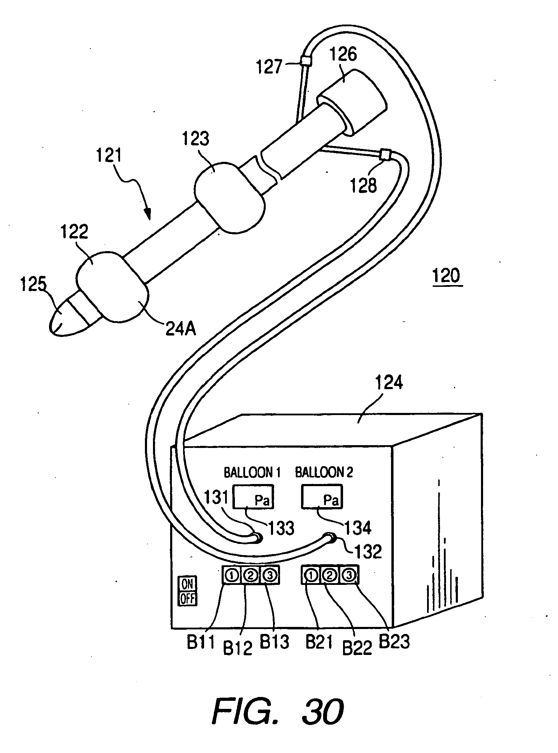

Fig. 30 shows a modification of the second embodiment. This modification is concerned with laterally organizing activated conditions of the respective balloons in the respective plural positional fixing patterns. In the second embodiment, plural balloons being activated are decided every positional fixing patterns and the preset is made such that the plural balloons are subjected to fixing to which target parts. In the present modification, the lateral organization gives three modes showing the positional fixing for each balloon. Thus, for each balloon, a single desired mode is selected from the three modes and the air supply is automatically controlled based on a combination of the selected modes.

A therapeutic system 120 in Fig. 30 is provided with an overtube 121 and a control box 124 which controls the air supply to first and second balloons 122 and 123 equipped on the overtube 121. The overtube 121 is equipped with a distal end portion 125 located at one end of the overtube, a grip 126 located at the other end thereof, and air-supply mouth rings 127 and 128 to send the air to the two balloons 122 and 123. The air-supply mouth rings 127 and 128 are connected to air-supply ports 131 and 132 of the control box 124 via tubes 129 and 130, respectively.

The control box 124 is also provided with three selection buttons B11, B12 and B13 selectively pressed for specifying a target part assigned to the first balloon 122 and further three selection buttons B21, B22 and B23 selectively pressed for specifying a target part assigned to the first balloon 123.

Of the three selection buttons B11, B12 and B13 for the first balloon 122,
- the button B11 is to supply a given amount of air for being fixed to the duodenum F5,
- the button B12 is to supply a given amount of air for being fixed to the duodenal bulb F4, and
- the button B13 is to supply a given amount of air for being fixed to the jejunum g1.

Further, of the three selection buttons B21, B22 and B23 for the second balloon 123,
- the button B21 is to supply a given amount of air for being fixed to the duodenal bulb F4,
- the button B22 is to supply a given amount of air for being fixed to the stomach (pylorus) F2, and
- the button B23 is to supply a given amount of air for being fixed to the jejunum g1.

Thus, an operator orally inserts the overtube 121, together with the insertion tube of an endoscope, into the object being examined, and then manually operates two selection buttons with the desired timing. The two selection buttons belong to a desired pattern among the three selection patterns given by a combination of the buttons B11 and B21, a combination of the buttons B21 and B22, and a combination of the buttons B13 and B23. That is, the first selection pattern is based on the combination of the buttons B11 and B21, the second selection pattern is based on the combination of the buttons B21 and B22, and the third selection pattern is based on the combination the buttons B13 and B23. Hence, for example, for selecting the first selection pattern, an operator first presses, for example, the button B11, and then presses the button B21. Incidentally, an interlock function can be added to the system, in which, when the button B11 for the first balloon 122 is pressed, the function is to invalidate the buttons (B22, B23) other than the button B21 as to the second balloon 123.

The control box 124 is provided with, on its front, pressure display monitors 133 and 134 to display pressures of the first and second balloons 122 and 123, a controller, and an air-supply device, like the foregoing second embodiment. Hence, for the fixing position selected by each button, a given amount of air which is preset is supplied to each of the first and second patterns 122 and 123. The internal pressures of the first and second balloons 122 and 123 are thus set to a given value. In addition, for every balloon and every fixing position, an amount of air to be sent, that is, the internal pressure may be changed. Alternatively, to make the internal pressures of all the balloons equal to each other, their internal volumes may be set to the same value.

The present modification also allows the overtube 121 to be positionally fixed to each organ in a reliable manner.

Incidentally, the scope of the present invention will not be limited to the configurations described in the foregoing embodiments and their various modifications, but the present invention may be reduced into practice in appropriate modes combined with conventional known structures, without departing from the scope of the present invention described in the appended claims.

## Claims

1. An overtube, comprising:
- a flexible tubular portion, in which an insertion channel is formed, an endoscope being insertable through the insertion channel; and
- a plurality of balloons located at plural positions on an outer surface of the flexible tubular portion in an axial direction thereof, the balloons being adapted to be expanded and shrunk selectively, being substantially equal in internal cubic volume to each other when being expanded, respectively, and including at least two balloons having mutually different lengths in the axial direction and being adapted to be fixed to an inner wall of either a body cavity or a tubular cavity of an object being examined at mutually different positions of the inner wall by expanding the balloons.

2. The overtube of claim 1, wherein the plurality of balloons includes a balloon adapted to be fixed to a duodenum of the object and a balloon adapted to be fixed to a duodenal bulb of the object, which are located in this order from a distal end portion of the flexible tubular portion.

3. The overtube of claim 1, wherein the plurality of balloons includes a balloon adapted to be fixed to a duodenum of the object and a balloon adapted to be fixed to a jejunum of the object, which are located in this order from a distal end portion of the flexible tubular portion.

4. The overtube of claim 1, wherein the plurality of balloons includes a balloon adapted to be fixed to a duodenal bulb of the object and a balloon adapted to be fixed to a stomach of the object, which are located in this order from a distal end portion of the flexible tubular portion.

5. The overtube of claim 1, wherein the plurality of balloons includes a balloon adapted to be fixed to a duodenum of the object, a balloon adapted to be fixed to a duodenal bulb of the object, and a balloon adapted to be fixed to a stomach of the object, which are located in this order from a distal end portion of the flexible tubular portion.

6. The overtube of claim 1, wherein the plurality of balloons includes a balloon adapted to be fixed to a duodenum of the object, a balloon adapted to be fixed to a duodenal bulb of the object, and a balloon adapted to be fixed to a jejunum of the object, which are located in this order from a distal end portion of the flexible tubular portion.

7. The overtube of claim 6, wherein
- a distance between the plurality of balloons in the axial direction is set such that the distance between the balloon adapted to be fixed to the duodenum and the balloon adapted to be fixed to the duodenal bulb is smaller than the distance between the balloon adapted to be fixed to the duodenal bulb and the balloon adapted to be fixed to the jejunum, and
- a diameter of each of the plurality of balloons in a direction perpendicular to the axial direction when being expanded is set such that the diameter of the balloon adapted to be fixed to the jejunum is smaller than the diameter of the balloon adapted to be fixed to the duodenum smaller than the diameter of the balloon adapted to be fixed to the duodenal bulb.

8. The overtube of claim 1, wherein the plurality of balloons includes a balloon adapted to be fixed to a duodenum of the object, a balloon adapted to be fixed to a duodenal bulb of the object, and a balloon adapted to be fixed to a stomach of the object, which are located in this order from a distal end portion of the flexible tubular portion.

9. The overtube of claim 8, wherein
- a distance between the plurality of balloons in the axial direction is set such that the distance between the balloon adapted to be fixed to the duodenal bulb and the balloon adapted to be fixed to the stomach is smaller than the distance between the balloon adapted to be fixed to the duodenum and the balloon adapted to be fixed to the duodenal bulb, and
- a diameter of each of the plurality of balloons in a direction perpendicular to the axial direction when being expanded is set such that the diameter of the balloon adapted to be fixed to the jejunum is smaller than the diameter of the balloon adapted to be fixed to the duodenal bulb smaller than the diameter of the balloon adapted to be fixed to the stomach.

10. The overtube of claim 1, wherein the plurality of balloons includes a balloon adapted to be fixed to a duodenum of the object, a balloon adapted to be fixed to a duodenal bulb of the object, a balloon adapted to be fixed to a stomach of the object, and a balloon adapted to be fixed to a jejunum of the object, which are located in this order from a distal end portion of the flexible tubular portion.

11. The overtube of claim 10, wherein
- a distance between the plurality of balloons in the axial direction is set such that the distance between the balloon adapted to be fixed to the duodenal bulb and the balloon adapted to be fixed to the stomach, the distance between the balloon adapted to be fixed to the duodenum and the balloon adapted to be fixed to the duodenal bulb, and the distance between the balloon adapted to be fixed to the duodenum and the balloon adapted to be fixed to the jejunum become larger in this order; and
- a diameter of each of the plurality of balloons in a direction perpendicular to the axial direction when being expanded is set such that the diameter of the balloon adapted to be fixed to the jejunum, the diameter of the balloon adapted to be fixed to the duodenum, the diameter of the balloon adapted to be fixed to the duodenal bulb, and the diameter of the balloon adapted to be fixed to the stomach become larger in this order.

12. The overtube of claim 1, wherein the plurality of balloons includes a balloon adapted to be fixed to a duodenum of the object, a balloon adapted to be fixed to a cardia of a stomach of the object, and a balloon adapted to be fixed to an esophagus of the object, which are located in this order from a distal end portion of the flexible tubular portion.

13. The overtube of claim 12, wherein
- a distance between the plurality of balloons in the axial direction is set such that the distance between the balloon adapted to be fixed to the stomach and the balloon adapted to be fixed to the esophagus is smaller than the distance between the balloon adapted to be fixed to the duodenum and the balloon adapted to be fixed to the stomach; and
- a diameter of each of the plurality of balloons in a direction perpendicular to the axial direction when being expanded is set such that the diameter of the balloon adapted to be fixed to the esophagus, the diameter of the balloon adapted to be fixed to the duodenum, and the diameter of the balloon adapted to be fixed to the stomach become larger in this order.

14. The overtube of claim 1, comprising
- a plurality of mouth rings located on the flexible tubular portion and used for supplying fluid to the plurality of balloons; and
- markers put on the plurality of mouth rings and formed to show a combination of selectively used balloons among the plurality of mouth rings, wherein common markers indicate possible positional fixing patterns of the balloons.

15. A therapeutic system comprising:
- an endoscope;
- an overtube according to any one of claims 1 to 14; and
- a controller that selectively controls the expansion of the plurality of balloons.

16. The therapeutic system of claim 15, wherein the controller comprises means that selectively combines at least two balloons among the plurality of balloons depending on a type of the body cavity or the tubular cavity and that makes the at least two balloons expand by supplying fluid to the balloons combination by combination, the at least two balloons belonging to a selected combination.

## Patentansprüche

1. Überschlauch, mit:
- einem flexiblen schlauchartigen Abschnitt, in dem ein Einführungskanal gebildet ist, durch den ein Endoskop einführbar ist; und
- einer Mehrzahl von Ballonen, die an mehreren Positionen an einer Außenoberfläche des flexiblen schlauchartigen Abschnitts in dessen axialer Richtung angeordnet sind, wobei die Ballone dazu ausgebildet sind, wahlweise expandiert oder geschrumpft zu werden, und ein im Wesentlichen gleiches Innenraumvolumen aufweisen, wenn sie expandiert sind, und mit zumindest zwei Ballonen, die in der axialen Richtung unterschiedliche Längen aufweisen und die dazu ausgebildet sind, an einer Innenwand eines Körperhohlraumes oder eines schlauchartigen Hohlraumes eines zu untersuchenden Objektes an voneinander unterschiedlichen Orten der Innenwand durch Expandieren der Ballone angebracht zu werden.

2. Überschlauch nach Anspruch 1, wobei die Mehrzahl von Ballonen einen Ballon, der dazu ausgebildet ist, am Duodenum des Objektes befestigt zu werden, und einen Ballon umfasst, der dazu ausgebildet ist, am Bulbus Duodeni des Objektes angeordnet zu werden, die in dieser Reihenfolge von einem distalen Endabschnitt des flexiblen schlauchförmigen Abschnittes angeordnet sind.

3. Überschlauch nach Anspruch 1, wobei die Mehrzahl von Ballonen einen Ballon, der dazu ausgebildet ist, am Duodenum des Objektes angeordnet zu werden, und einen Ballon umfasst, der dazu ausgebildet ist, am Jenunum des Objektes angeordnet zu werden, die in dieser Reihenfolge von einem distalen Endabschnitt des flexiblen schlauchartigen Abschnittes angeordnet sind.

4. Überschlauch nach Anspruch 1, wobei die Mehrzahl von Ballonen einen Ballon, der dazu ausgebildet ist, am Bulbus Duodeni des Objektes angeordnet zu werden, und einen Ballon umfasst, der dazu ausgebildet ist, am Magen des Objektes angeordnet zu werden, die in dieser Reihenfolge von einem distalen Endabschnitt des flexiblen schlauchförmigen Abschnittes angeordnet sind.

5. Überschlauch nach Anspruch 1, wobei die Mehrzahl von Ballonen einen Ballon, der dazu ausgebildet ist, am Duodenum des Objekts angeordnet zu werden, einen Ballon, der dazu ausgebildet ist, am Bulbus Duodeni des Objektes angeordnet zu werden, und einen Ballon umfasst, der dazu ausgebildet ist, am Magen des Objektes
angeordnet zu werden, die in dieser Reihenfolge von einem distalen Endabschnitt des flexiblen schlauchartigen Abschnittes angeordnet sind.

6. Überschlauch nach Anspruch 1, wobei die Mehrzahl von Ballonen einen Ballon, der dazu ausgebildet ist, am Duodenum des Objektes angeordnet zu werden, einen Ballon, der dazu ausgebildet ist, am Bulbus Duodeni des Objektes angeordnet zu werden, und einen Ballon umfasst, der dazu ausgebildet ist, am Jenumum des Objektes angeordnet zu werden, die in dieser Reihenfolge von einem distalen Endabschnitt des flexiblen schlauchförmigen Abschnittes angeordnet sind.

7. Überschlauch nach Anspruch 6, wobei
- ein Abstand zwischen der Mehrzahl von Ballonen in der axialen Richtung so festgelegt ist, dass der Abstand zwischen dem Ballon, der dazu ausgebildet ist, am Duodenum angeordnet zu werden, und dem Ballon, der dazu ausgebildet ist, am Bulbus Duodeni angeordnet zu werden, kleiner ist als der Abstand zwischen dem Ballon, der dazu ausgebildet ist, am Bulbus Duodeni angeordnet zu werden, und dem Ballon, der dazu ausgebildet ist, am Jenunum angeordnet zu werden, und
- ein Durchmesser eines jeden der Mehrzahl von Ballonen in einer Richtung rechtwinklig zur axialen Richtung, wenn er expandiert ist, so festgelegt ist, dass der Durchmesser des Ballons, der dazu ausgebildet ist, am Jenunum angeordnet zu werden, kleiner ist als der Durchmesser des Ballons, der dazu ausgebildet ist, am Duodenum angeordnet zu werden, der kleiner ist als der Durchmesser des Ballons, der dazu ausgebildet ist, am Bulbus Duodeni angeordnet zu werden.

8. Überschlauch nach Anspruch 1, wobei die Mehrzahl von Ballonen einen Ballon, der dazu ausgebildet ist, am Duodenum des Objektes angeordnet zu werden, einen Ballon, der dazu ausgebildet ist, am Bulbus Duodeni des Objektes angeordnet zu werden, und einen Ballon umfasst, der dazu ausgebildet ist, am Magen des Objektes angeordnet zu werden, die in dieser Reihenfolge von einem distalen Endabschnitt des flexiblen schlauchartigen Abschnittes angeordnet sind.

9. Überschlauch nach Anspruch 8, wobei
- ein Abstand zwischen der Mehrzahl von Ballonen in der axialen Richtung so festgelegt ist, dass der Abstand zwischen dem Ballon, der dazu ausgebildet ist, am Bulbus Duodeni angeordnet zu werden, und dem Ballon, der dazu ausgebildet ist, am Magen angeordnet zu werden, kleiner ist als der Abstand zwischen dem Ballon, der dazu ausgebildet ist, am Duodenum angeordnet zu werden, und dem Ballon, der dazu ausgebildet ist, am Bulbus Duodeni angeordnet zu werden, und
- ein Durchmesser eines jeden der Mehrzahl von Ballonen in einer Richtung rechtwinklig zur axialen Richtung, wenn sie expandiert sind, so festgelegt ist, dass der Durchmesser des Ballons, der dazu ausgebildet ist, am Jenunum angeordnet zu werden, kleiner ist als der Durchmesser des Ballons, der dazu ausgebildet ist, am Bulbus Duodeni angeordnet zu werden, der kleiner ist als der Durchmesser des Ballons, der dazu ausgebildet ist, am Magen angeordnet zu werden.

10. Überschlauch nach Anspruch 1, wobei die Mehrzahl von Ballonen einen Ballon, der dazu ausgebildet ist, am Duodenum des Objektes angeordnet zu werden, einen Ballon, der dazu ausgebildet ist, am Bulbus Duodeni des Objektes angeordnet zu werden, einen Ballon, der dazu ausgebildet ist, am Magen des Objektes angeordnet zu werden, und einen Ballon umfasst, der dazu ausgebildet ist, am Jenunum des Objektes angeordnet zu werden, die in dieser Reihenfolge von einem distalen Endabschnitt des flexiblen schlauchförmigen Abschnittes angeordnet sind.

11. Überschlauch nach Anspruch 10, wobei
- ein Abstand zwischen der Mehrzahl von Ballonen in der axialen Richtung so festgelegt ist, dass der Abstand zwischen dem Ballon, der dazu ausgebildet ist, am Bulbus Duodeni angeordnet zu werden, und dem Ballon, der dazu ausgebildet ist, am Magen angeordnet zu werden, der Abstand zwischen dem Ballon, der dazu ausgebildet ist, am Duodenum angeordnet zu werden, und dem Ballon, der dazu ausgebildet ist, am Bulbus Duodeni angeordnet zu werden, und der Abstand zwischen dem Ballon der dazu ausgebildet ist, am Duodenum angeordnet zu werden, und dem Ballon, der dazu ausgebildet ist, am Jenunum angeordnet zu werden, in dieser Reihenfolge größer werden;
- ein Durchmesser eines jeden der Mehrzahl von Ballonen in einer Richtung rechtwinklig zur axialen Richtung, wenn sie expandiert sind, so festgelegt ist, dass der Durchmesser des Ballons, der dazu ausgebildet ist, am Jenunum angeordnet zu werden, der Durchmesser des Ballons, der dazu ausgebildet ist, am Duodenum angeordnet zu werden, der Durchmesser des Ballons, der dazu ausgebildet ist, am Bulbus Duodeni angeordnet zu werden, und der Durchmesser des Ballons, der dazu ausgebildet ist, am Magen angeordnet zu werden, in dieser Reihenfolge größer werden.

12. Überschlauch nach Anspruch 1, wobei die Mehrzahl von Ballonen einen Ballon, der dazu ausgebildet ist, am Duodenum des Objektes angeordnet zu werden, einen Ballon der dazu ausgebildet ist, an der Cardia des Magens des Objektes angeordnet zu werden, und einen Ballon umfasst, der dazu ausgebildet ist, am Esophagus des
Objektes angeordnet zu werden, die in dieser Reihenfolge von einem distalen Endabschnitt des flexiblen schlauchförmigen Abschnittes angeordnet sind.

13. Überschlauch nach Anspruch 12, wobei
- ein Abstand zwischen der Mehrzahl von Ballonen in der axialen Richtung so festgelegt ist, dass der Abstand zwischen dem Ballon, der dazu ausgebildet ist, am Magen angeordnet zu werden, und dem Ballon, der dazu ausgebildet ist, am Esophargus angeordnet zu werden, kleiner ist, als der Abstand zwischen dem Ballon, der dazu ausgebildet ist, am Duodenum angeordnet zu werden, und dem Ballon, der dazu ausgebildet ist, am Magen angeordnet zu werden; und
- ein Durchmesser eines jeden der Mehrzahl von Ballonen in einer Richtung rechtwinklig zur axialen Richtung, wenn sie expandiert sind, so festgelegt ist, dass der Durchmesser des Ballons, der dazu ausgebildet ist, am Esophargus angeordnet zu werden, der Durchmesser des Ballons, der dazu ausgebildet ist, am Duodenum angeordnet zu werden, und der Durchmesser des Ballons, der dazu ausgebildet ist, am Magen angeordnet zu werden, in dieser Reihenfolge größer werden.

14. Überschlauch nach Anspruch 1, mit
- einer Mehrzahl von Mundringen, die am flexiblen schlauchartigen Abschnitt angeordnet sind und die zum Zuführen von Fluid zur Mehrzahl von Ballonen verwendet werden; und
- Markierungen, die an der Mehrzahl von Mundringen aufgebracht sind und dazu ausgebildet sind, eine Kombination von wahlweise verwendeten Ballonen aus der Mehrzahl von Mundringen anzuzeigen, wobei gemeinsame Markierungen mögliche Positionsbefestigungsmuster der Ballone anzeigen.

15. Therapeutisches System, mit:
- einem Endoskop;
- einem Überschlauch nach einem der Ansprüche 1 bis 14; und
- einer Steuerungseinrichtung, die wahlweise das Expandieren der Mehrzahl von Ballonen steuert.

16. Therapeutisches System nach Anspruch 15, wobei die Steuerungseinrichtung eine Einrichtung aufweist, die wahlweise zumindest zwei Ballone aus der Mehrzahl von Ballonen in Abhängigkeit eines Typs des Körperhohlraumes oder des schlauchförmigen Hohlraumes kombiniert und die bewirkt, dass zumindest zwei Ballone durch Zuführen von Fluid zu der Ballonkombination und zwar Kombination für Kombination expandiert werden, wobei zumindest zwei Ballone einer ausgewählten Kombination angehören.

## Revendications

1. Sur-tube, comprenant
- une partie tubulaire flexible, dans laquelle un canal d'insertion est formé, un endoscope pouvant être inséré à travers le canal d'insertion ; et
- une pluralité de ballonnets situés en des positions multiples sur une surface extérieure de la partie tubulaire flexible dans une direction axiale de celle-ci, les ballonnets étant adaptés à être gonflés et dégonflés sélectivement, étant substantiellement égaux en volume cubique interne les uns aux autres lorsqu'ils sont gonflés, respectivement, et incluant au moins deux ballonnets ayant des longueurs mutuellement différentes dans la direction axiale et étant adaptés à être fixés à une paroi interne soit d'une cavité corporelle, soit d'une cavité tubulaire d'un objet étant examiné en des positions mutuellement différentes de la paroi interne en gonflant les ballonnets.

2. Sur-tube selon la revendication 1, dans lequel la pluralité de ballonnets inclut un ballonnet adapté à être fixé à un duodénum de l'objet et un ballonnet adapté à être fixé à un bulbe duodénal de l'objet, qui sont situés dans cet ordre à partir d'une partie d'extrémité distale de la partie tubulaire flexible.

3. Sur-tube selon la revendication 1, dans lequel la pluralité de ballonnets inclut un ballonnet adapté à être fixé à un duodénum de l'objet et un ballonnet adapté à être fixé à un jéjunum de l'objet, qui sont situés dans cet ordre à partir d'une partie d'extrémité distale de la partie tubulaire flexible.

4. Sur-tube selon la revendication 1, dans lequel la pluralité de ballonnets inclut un ballonnet adapté à être fixé à un bulbe duodénal de l'objet et un ballonnet adapté à être fixé à un estomac de l'objet, qui sont situés dans cet ordre à partir d'une partie d'extrémité distale de la partie tubulaire flexible.

5. Sur-tube selon la revendication 1, dans lequel la pluralité de ballonnets inclut un ballonnet adapté à être fixé à un duodénum de l'objet, un ballonnet adapté à être fixé à un bulbe duodénal de l'objet et un ballonnet adapté à être fixé à un estomac de l'objet, qui sont situés dans cet ordre à partir d'une partie d'extrémité distale de la partie tubulaire flexible.

6. Sur-tube selon la revendication 1, dans lequel la pluralité de ballonnets inclut un ballonnet adapté à être fixé à un duodénum de l'objet, un ballonnet adapté à être fixé à un bulbe duodénal de l'objet et un ballonnet adapté à être fixé à un jéjunum de l'objet, qui sont situés dans cet ordre à partir d'une partie d'extrémité distale de la partie tubulaire flexible.

7. Sur-tube selon la revendication 6, dans lequel
- une distance entre la pluralité de ballonnets dans la direction axiale est fixée de telle sorte que la distance entre le ballonnet adapté à être fixé au duodénum et le ballonnet adapté à être fixé au bulbe duodénal est plus petite que la distance entre le ballonnet adapté à être fixé au bulbe duodénal et le ballonnet adapté à être fixé au jéjunum, et
- un diamètre de chacun de la pluralité de ballonnets dans une direction perpendiculaire à la direction axiale lorsqu'ils sont gonflés est fixé de telle sorte que le diamètre du ballonnet adapté à être fixé au jéjunum est plus petit que le diamètre du ballonnet adapté à être fixé au duodénum plus petit que le diamètre du ballonnet adapté à être fixé au bulbe duodénal.

8. Sur-tube selon la revendication 1, dans lequel la pluralité de ballonnets inclut un ballonnet adapté à être fixé à un duodénum de l'objet, un ballonnet adapté à être fixé à un bulbe duodénal de l'objet et un ballonnet adapté à être fixé à un estomac de l'objet, qui sont situés dans cet ordre à partir d'une partie d'extrémité distale de la partie tubulaire flexible.

9. Sur-tube selon la revendication 8, dans lequel
- une distance entre la pluralité de ballonnets dans la direction axiale est fixée de telle sorte que la distance entre le ballonnet adapté à être fixé au bulbe duodénal et le ballonnet adapté à être fixé à l'estomac est plus petite que la distance entre le ballonnet adapté à être fixé au duodénum et le ballonnet adapté à être fixé au bulbe duodénal, et
- un diamètre de chacun de la pluralité de ballonnets dans une direction perpendiculaire à la direction axiale lorsqu'ils sont gonflés est fixé de telle sorte que le diamètre du ballonnet adapté à être fixé au jéjunum est plus petit que le diamètre du ballonnet adapté à être fixé au bulbe duodénal plus petit que le diamètre du ballonnet adapté à être fixé à l'estomac.

10. Sur-tube selon la revendication 1, dans lequel la pluralité de ballonnets inclut un ballonnet adapté à être fixé à un duodénum de l'objet, un ballonnet adapté à être fixé à un bulbe duodénal de l'objet, un ballonnet adapté à être fixé à un estomac de
l'objet et un ballonnet adapté à être fixé à un jéjunum de l'objet, qui sont situés dans cet ordre à partir d'une partie d'extrémité distale de la partie tubulaire flexible.

11. Sur-tube selon la revendication 10, dans lequel
- une distance entre la pluralité de ballonnets dans la direction axiale est fixée de telle sorte que la distance entre le ballonnet adapté à être fixé au bulbe duodénal et le ballonnet adapté à être fixé à l'estomac, la distance entre le ballonnet adapté à être fixé au duodénum et la distance entre le ballonnet adapté à être fixé au bulbe duodénal, et la distance entre le ballonnet adapté à être fixé au duodénum et le ballonnet adapté à être fixé au jéjunum deviennent plus grandes dans cet ordre ; et
- un diamètre de chacun de la pluralité de ballonnets dans une direction perpendiculaire à la direction axiale lorsqu'ils sont gonflés, est fixé de telle sorte que le diamètre du ballonnet adapté à être fixé au jéjunum, le diamètre du ballonnet adapté à être fixé au duodénum, le diamètre du ballonnet adapté à être fixé au bulbe duodénal et le diamètre du ballonnet adapté à être fixé à l'estomac deviennent plus grands dans cet ordre.

12. Sur-tube selon la revendication 1, dans lequel la pluralité de ballonnets inclut un ballonnet adapté à être fixé à un duodénum de l'objet, un ballonnet adapté à être fixé à un cardia d'un estomac de l'objet, et un ballonnet adapté à être fixé à un oesophage de l'objet, qui sont situés dans cet ordre à partir d'une partie d'extrémité distale de la partie tubulaire flexible.

13. Sur-tube selon la revendication 12, dans lequel
- une distance entre la pluralité de ballonnets dans la direction axiale est fixée de telle sorte que la distance entre le ballonnet adapté à être fixé à l'estomac et le ballonnet adapté à être fixé à l'oesophage est plus petite que la distance entre le ballonnet adapté à être fixé au duodénum et le ballonnet adapté à être fixé à l'estomac ; et
- un diamètre de chacun de la pluralité de ballonnets dans une direction perpendiculaire à la direction axiale lorsqu'ils sont gonflés, est fixé de telle sorte que le diamètre du ballonnet adapté à être fixé à l'oesophage, le diamètre du ballonnet adapté à être fixé au duodénum et le diamètre du ballonnet adapté à être fixé à l'estomac deviennent plus grands dans cet ordre.

14. Sur-tube selon la revendication 1, comprenant
- une pluralité de bagues d'embouchure situées sur la partie tubulaire flexible et utilisées pour amener un fluide jusqu'à la pluralité de ballonnets ; et
- des marqueurs disposés sur la pluralité de bagues d'embouchure et formés de façon à montrer une combinaison de ballonnets sélectivement utilisés parmi la pluralité de bagues d'embouchure, dans lequel des marqueurs communs indiquent des motifs possibles de position de fixation des ballonnets.

15. Système thérapeutique comprenant :
- un endoscope ;
- un sur-tube selon l'une quelconque des revendications 1 à 14 ; et
- un contrôleur qui commande sélectivement le gonflage de la pluralité de ballonnets.

16. Système thérapeutique selon la revendication 15, dans lequel le contrôleur comprend un moyen qui combine sélectivement au moins deux ballonnets parmi la pluralité de ballonnets en fonction d'un type de la cavité corporelle ou de la cavité tubulaire et qui fait gonfler les au moins deux ballonnets en amenant un fluide jusqu'aux ballonnets combinaison par combinaison, les au moins deux ballonnets appartenant à une combinaison sélectionnée.
